(19) Europäisches Patentamt
European Patent Office
Office européen des brevets

(11) Numéro de publication : 0 289 430 B1

(12) **FASCICULE DE BREVET EUROPEEN**

(45) Date de publication du fascicule du brevet : **08.04.92 Bulletin 92/15**

(51) Int. Cl.$^5$ : **A23L 1/236**

(21) Numéro de dépôt : **88420012.2**

(22) Date de dépôt : **14.01.88**

(54) **Edulcorants dérivés des acides guanidinoacétique et éthanamidinoacétique; leurs applications; compositions contenant ces édulcorants.**

(30) Priorité : **15.01.87 FR 8700539**

(43) Date de publication de la demande : **02.11.88 Bulletin 88/44**

(45) Mention de la délivrance du brevet : **08.04.92 Bulletin 92/15**

(84) Etats contractants désignés : **AT BE CH DE ES FR GB GR IT LI LU NL SE**

(56) Documents cités :
**EP-A- 0 107 597**
**EP-A- 0 195 730**
**EP-A- 0 195 731**
**JOURNAL OF AGRICULTURAL AND FOOD CHEMISTRY, vol. 28, no. 6, novembre/décembre 1980, pages 1338-1340, American Chemical Society, Washington, D.C., US; K. KAWASHIMA et al.: "An intensely sweet analogue of kynurenine: 3-(4-chloroanthraniloyl)-DL-alanine"**

(56) Documents cités :
**JOURNAL OF AGRICULTURAL AND FOOD CHEMISTRY, vol. 21, no. 1, janvier/février 1973, pages 33-34, American Chemical Society, Washington, D.C., US; J.W. FINLEY et al.: "New sweetening agents: N'-formyl- and N'-acetylkynurenine"**

(73) Titulaire : **UNIVERSITE CLAUDE BERNARD - LYON 1**
**43, boulevard du 11 Novembre 1918**
**F-69622 Villeurbanne Cédex (FR)**

(72) Inventeur : **Nofre, Claude**
**119 cours Albert Thomas**
**F-69003 Lyon (FR)**
Inventeur : **Tinti, Jean Marie**
**5 avenue de Grenoble**
**F-69330 Meyzieu (FR)**
Inventeur : **Ouar, Farroudja**
**épouse Chatzopoulos 14, rue Raoul Follereau**
**F-42100 Saint Etienne (FR)**

(74) Mandataire : **Portal, Gérard et al**
**Cabinet Beau de Loménie 55, rue d'Amsterdam**
**F-75008 Paris (FR)**

EP 0 289 430 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux agents édulcorants utiles, notamment, pour édulcorer les aliments, les boissons, les confiseries, les pâtisseries, le chewing gum, les produits d'hygiène, les cosmétiques, les articles de toilette, les produits pharmaceutiques et vétérinaires. Elle concerne aussi des préparations et des compositions contenant de tels agents édulcorants.

Dans la demande de brevet européen EP-A-O 195 730 du Demandeur, on a décrit, comme nouveaux agents édulcorants, des composés de formule :

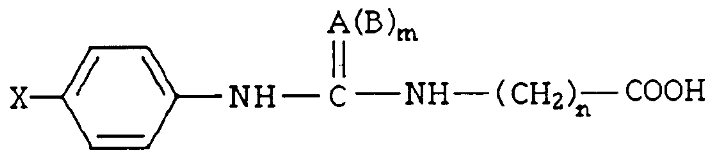

dans laquelle :

- A est :
  - N et
  - C ;
- m et n sont 1 ou 2 ;
- B est,
  - . quand n = 1 :
    - H,
    - CN,
    - $NO_2$,
    - $OCH_3$,
    - SOR,
    - $SO_2R$,
    - $SO_2NHR$ et
    - $SO_2N(R)_2$, R étant un groupe alkyle, cycloalkyle ou aryle ayant jusqu'à 10 atomes de carbone, 1 ou 2 atomes de carbone pouvant être remplacés par 1 ou 2 atomes de soufre ou d'oxygène,
  - . quand n = 2 :
    - H,
    - CN et
    - $OCH_3$ ;
- X, qui est en position 4, est :
  - CN et
  - $NO_2$, quand B est H, CN et $OCH_3$,
  - et est :
  - H,
  - $CF_3$,
  - CHO,
  - Cl,
  - CN,
  - $COCH_3$,
  - F et
  - $NO_2$, quand B est $NO_2$, SOR, $SO_2R$, $SO_2NHR$ et $SO_2N(R)_2$.

Dans la demande de brevet européen EP-A-0241 395 du Demandeur, on a proposé comme nouveaux agents édulcorants des composés de formule générale :

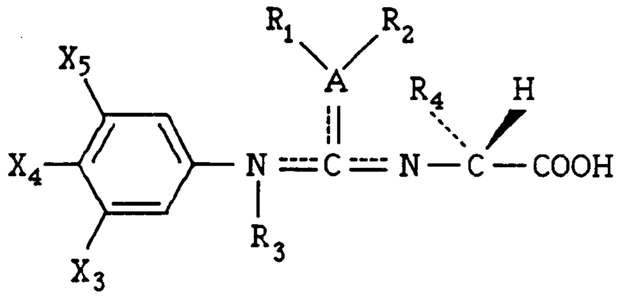

– dans laquelle $X_3$, $X_4$ et $X_5$ sont choisis dans le groupe comprenant :

H,
Br,
$CF_3$,
$CF_2CF_3$,
$CH_2CF_3$,
$C_1$-$C_4$ alkyl,
$CH=NOCH_3$,
CH=NOH,
CHO,
$CH_2OCH_3$,
$CH_2OH$,
Cl,
CN,
$COCF_3$,
$COC_1$-$C_3$ alkyl,
$CONH_2$,
$CONHC_1$-$C_3$ alkyl,
$CON(C_1$-$C_3$ alkyl$)_2$,
$COOC_1$-$C_3$ alkyl,
COOH,
F,
I,
$NH_2$,
$NHC_1$-$C_3$ alkyl,
$N(C_1$-$C_3$ alkyl$)_2$,
NHCHO,
$NHCOCH_3$,
$NHCONH_2$,
$NHSO_2CH_3$,
$NO_2$,
$OC_1$-$C_3$ alkyl,
$OCOCH_3$,
OH,
$SC_1$-$C_3$ alkyl,
$SOC_1$-$C_3$ alkyl,
$SO_2C_1$-$C_3$ alkyl,
$SO_2NH_2$,
$SO_2NHC_1$-$C_3$ alkyl,
$SO_2N(C_1$-$C_3$ alkyl$)_2$ et
$SO_3H$ ;

– dans laquelle A est choisi dans le groupe comprenant N et C ;

– dans laquelle $R_1$ est un atome d'hydrogène, ou un groupe hydrocarboné ou un groupe hydrocarboné modifié ayant jusqu'à 4 atomes de carbone, saturé ou insaturé, acyclique, cyclique ou mixte, et dans lequel, dans le groupe hydrocarboné modifié :

. 1 à 2 atomes de carbone peuvent être remplacés par 1 à 2 hétéroatomes, identiques ou différents, choisis dans le groupe comprenant les atomes d'azote, oxygène, soufre, chlore, brome et iode,

. et 1 à 3 atomes d'hydrogène peuvent être remplacés par 1 à 3 atomes de fluor ;

– dans laquelle $R_2$ est un groupe hydrocarboné ou un groupe hydrocarboné modifié, de 2 à 13 atomes de carbone, saturé ou insaturé, acyclique, cyclique ou mixte, et dans lequel, dans le groupe hydrocarboné modifié :

. 1 à 4 atomes de carbone peuvent être remplacés par 1 à 4 hétéroatomes, identiques ou différents, choisis dans le groupe comprenant les atomes d'azote, oxygène, soufre, chlore, brome et iode,

. et 1 à 5 atomes d'hydrogène peuvent être remplacés par 1 à 5 atomes de fluor ;

– dans laquelle $R_1$ et $R_2$ peuvent être fusionnés ;

– dans laquelle $R_3$ est choisi dans le groupe comprenant un atome d'hydrogène ou un groupe alkyle $C_1$-$C_3$ ;

– dans laquelle $R_4$ est choisi dans le groupe comprenant :

H et

$CH_3$ ;

à condition, quand $X_3$, $X_5$, $R_3$ et $R_4$ sont des atomes d'hydrogène,

. et quand $X_4$ est CN ou $NO_2$, que $R_1$ et $R_2$ ne soient pas CN ou $OCH_3$,

. et quand $X_4$ est H, $CF_3$, CHO, Cl, CN, $COCH_3$, F ou $NO_2$, que $R_1$ ne soit pas $NO_2$ ou $SOC_1$-$C_2$ alkyl et que $R_2$ ne soit pas $NO_2$, SOR, $SO_2R$, $SO_2NHR$ et $SO_2N(R)_2$, R étant un groupe alkyle, cycloalkyle ou aryle ayant jusqu'à 10 atomes de carbone, 1 ou 2 de ces atomes de carbone pouvant être remplacés par 1 ou 2 atomes de soufre ou d'oxygène.

Les agents édulcorants de la présente invention répondent à la formule générale :

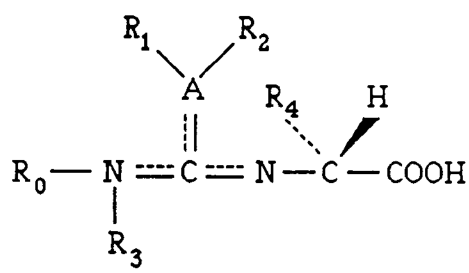

en incluant les formes tautomères et les sels physiologiquement acceptables,

– dans laquelle A est choisi dans le groupe comprenant N et C ;

– dans laquelle $R_1$ est un atome d'hydrogène ou un groupe hydrocarboné ou un groupe hydrocarboné modifié, ayant jusqu'à 4 atomes de carbone, saturé ou insaturé, acyclique, cyclique ou mixte, et dans lequel, dans le groupe hydrocarboné modifié :

. 1 à 2 atomes de carbone peuvent être remplacés par 1 à 2 hétéroatomes, identiques ou différents, choisis dans le groupe comprenant les atomes d'azote, oxygène, soufre, chlore, brome et iode,

. et 1 à 3 atomes d'hydrogène peuvent etre remplacés par 1 à 3 atomes de fluor ;

– dans laquelle $R_2$ est un groupe hydrocarboné ou un groupe hydrocarboné modifié, de 2 à 13 atomes de carbone, saturé ou insaturé, acyclique, cyclique ou mixte, et dans lequel, dans le groupe hydrocarboné modifié :

– 1 à 4 atomes de carbone peuvent être remplacés par 1 à 4 hétéroatomes, identiques ou différents, choisis dans le groupe comprenant les atomes d'azote, oxygène, soufre, chlore, brome et iode,

– et 1 à 5 atomes d'hydrogène peuvent être remplacés par 1 à 5 atomes de fluor ;

– dans laquelle $R_1$ et $R_2$ peuvent être fusionnés ;

– dans laquelle $R_3$ est choisi dans le groupe comprenant un atome d'hydrogène ou un groupe alkyle $C_1$-$C_3$ ;

– dans laquelle $R_4$ est choisi dans le groupe comprenant :

H et

$CH_3$.

Ces nouveaux édulcorants se caractérisent en ce que $R_o$ est choisi dans le groupe comprenant :

. les groupes hétérocycliques dérivés des groupes parents hydrocarbonés 1,3-cyclopentadièn-1-yle, 2,4-cyclopentadièn-1-yle et phényle par remplacement de 1 à 3 atomes de carbone du cycle parent hydrocarboné par 1 à 3 atomes d'azote, d'oxygène et de soufre,

et dans lesquels les atomes d'hydrogène des positions 3 et 4 des groupes parents 1,3-cyclopentadièn-1-yle, 2,4-cyclopentadièn-1-yle et des positions 3, 4 et 5 du groupe parent phényle peuvent être substitués dans le groupe hétérocyclique correspondant par Br, $CF_3$, $CH_3$, Cl, CN, F, I, $NH_2$, $NO_2$, $OCH_3$ ou OH,

. les groupes carbocycliques indan-5-yle, indèn-2-yle, indèn-5-yle, indèn-6-yle et les groupes hétérocycliques correspondants obtenus par remplacement de 1 à 4 atomes de carbone du cycle parent hydrocarboné

par 1 à 4 atomes d'azote, d'oxygène ou de soufre, ou par les groupes SO, CO et $SO_2$ pour les carbones 1 et 3 du groupe parent indan-5-yle ou par les groupes NO ou $CCH_3$ pour le carbone 3 du groupe parent 1*H*-indèn-5-yle ou pour les carbones 1 et 3 du groupe parent 2*H*-indèn-5 yle,

et dans lesquels l'atome d'hydrogène de la position 7 du groupe indan(ou indèn)-5-yle et de la position 4 du groupe indèn-6-yle peut être substitué dans le groupe carbocyclique et dans le groupe hétérocyclique correspondant par Br, $CF_3$, $CH_3$, Cl, CN, F, I, $NH_2$, $NO_2$, $OCH_3$ ou OH,

. les groupes carbocycliques 2-naphtyle, 5,6,7,8-tétrahydro-2-naphtyle et les groupes hétérocycliques correspondants obtenus par remplacement de 1 à 4 atomes de carbone du cycle parent hydrocarboné par 1 à 4 atomes d'azote, d'oxygène, de soufre et par les groupes SO, CO et $SO_2$ pour les carbones 5 et 8 du groupe parent 5,6,7,8-tétrahydronaphtyle,

et dans lesquels l'atome d'hydrogène de la position 4 des groupes 2-naphtyle et 5,6,7,8-tétrahydro-2-naphtyle peut être substitué, aussi bien dans le groupe carbocyclique que dans le groupe hétérocyclique correspondant, par Br, $CF_3$, $CH_3$, Cl, CN, F, I, $NH_2$, $NO_2$, $OCH_3$, OH ;

– à condition, quand A = N, que $R_2$ ne soit pas un groupe

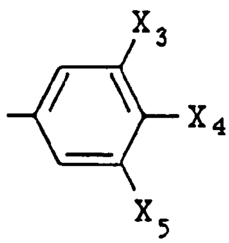

dans lequel $X_3$, $X_4$ et $X_5$ sont :

H,
Br,
$CF_3$,
$CF_2CF_3$,
$CH_2CF_3$,
$C_1$-$C_4$ alkyl,
$CH=NOCH_3$,
CH=NOH,
CHO,
$CH_2OCH_3$,
$CH_2OH$,
Cl,
CN,
$COCF_3$,
$COC_1$-$C_3$ alkyl,
$CONH_2$,
$CONHC_1$-$C_3$ alkyl,
$CON(C_1$-$C_3$ alkyl$)_2$,
$COOC_1$-$C_3$ alkyl,
COOH,
F,
I,
$NH_2$,
$NHC_1$-$C_3$ alkyl,
$N(C_1$-$C_3$ alkyl$)_2$,
NHCHO,
$NHCOCH_3$,
$NHCONH_2$,
$NHSO_2CH_3$,
$NO_2$,
$OC_1$-$C_3$ alkyl,
$OCOCH_3$,
OH,

$SC_1$-$C_3$ alkyl,
$SOC_1$-$C_3$ alkyl,
$SO_2C_1$-$C_3$ alkyl,
$SO_2NH_2$,
$SO_2NHC_1$-$C_3$ alkyl,
$SO_2N(C_1$-$C_3$ alkyl$)_2$ et
$SO_3H$.

Avantageusement en pratique :

– les hétéroatomes qui, dans les cycles parents hydrocarbonés remplacent les atomes de carbone pour former un groupe hétérocyclique $R_o$ correspondant sont :
. N pour remplacer CH, et
. NH, O, S, SO et $SO_2$ pour remplacer $CH_2$ ;

– $R_o$, quand $R_o$ est un groupe hétérocyclique, est choisi dans le groupe comprenant un cycle pyrrolyle, pyrazolyle, imidazolyle, furyle, oxazolyle, isoxazolyle, oxadiazolyle, furazanyle, thiényle, thiazolyle, isothiazolyle, pyridyle, 2-cyanopyridyle, pyridazinyle, pyrimidinyle, 2-cyanopyrimidinyle, pyrazinyle, indolyle, isoindolyle, indolizinyle, indazolyle, purinyle, isobenzofuranyle, 3,4-méthylènedioxyphényle, benzisoxazolyle, benzofurazanyle, benzothiazolyle, saccharin-5-yle, saccharin-6-yle, quinolyle, isoquinolyle, cinnolinyle, phtalazinyle, quinazolinyle, quinoxalinyle, naphtyridinyle, 1,5-naphtyridinyle, 1,6-naphtyridinyle, 1,7-naphtyridinyle, 1,3,5-triazanaphtyle, 1,4,5-triazanaphtyle, 1,2,8-triazanaphtyle, 1,3,8-triazanaphtyle, 1,2,5-triazanaphtyle, ptéridinyle ou isocoumarinyle ;

– A est un atome d'azote ;

– $R_1$ est choisi dans le groupe constitué par H et $CH_3$ ;

– $R_2$ est choisi dans le groupe constitué par:
$C_2$-$C_{13}$ alk(én)(yn)yl normal,
$C_3$-$C_{13}$ alk(én)(yn)yl ramifié,
$C_3$-$C_{13}$ cycloalk(én)yl,
$C_4$-$C_{13}$ alk(én)yl cycloalk(én)yl,
$C_4$-$C_{13}$ cycloalk(én)yl alk(én)yl,
$C_5$-$C_{13}$ alk(én)yl cycloalk(én)yl alk(én)yl,
$C_7$-$C_{13}$ alk(én)yl bicycloalk(én)yl,
$C_7$-$C_{13}$ bicycloalk(én)yl fusionné,
$C_8$-$C_{13}$ alk(én)yl bicycloalk(én)yl fusionné,
$C_8$-$C_{13}$ bicycloalk(én)yl fusionné alk(én)yl,
$C_9$-$C_{13}$ alk(én)yl bicycloalk(én)yl fusionné alk(én)yl,
$C_{10}$-$C_{13}$ tricycloalk(én)yl fusionné,
$C_{11}$-$C_{13}$ alk(én)yl tricycloalk(én)yl fusionné,
$C_{11}$-$C_{13}$ tricycloalk(én)yl fusionné alk(én)yl,
et $C_{13}$ alk(én)yl tricycloalk(én)yl fusionné alk(én)yl ;

. dans lesquels jusqu'à 4 atomes de carbone peuvent être remplacés par des hétéroatomes, identiques ou différents, choisis dans le groupe constitué par :
N pour remplacer CH,
NH, O et S pour remplacer $CH_2$, et
Cl, Br et I pour remplacer $CH_3$,

. et dans lesquels jusqu'à 5 atomes d'hydrogène peuvent être substitués par des atomes de fluor ;

– $R_3$, dans l'une des formes tautomères de la molécule, est un atome d'hydrogène ;

– $R_4$ est un atome d'hydrogène.

Dans une forme d'exécution préférée, les agents édulcorants selon l'invention consistent en des composés de formule :

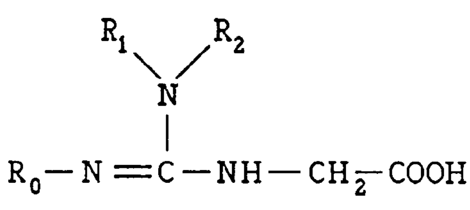

dans laquelle :

– $R_o$ est choisi dans le groupe constitué par un groupe 2-cyanopyrid-5-yle, 2-cyanopyrimidin-5-yle, indazol-6-yle, benzisoxazol-5-yle, benzisoxazol-6-yle, benzofurazan-5-yle, isoquinol-6-yle, quinazolin-7-yle, 1,7-naphtyridin-3-yle ou 1,3,5-triazanapht-7-yle,

– $R_1$ est H ou $CH_3$,

– $R_2$ est *c*-$C_8H_{15}$, $CH_2C_6H_5$, $CH_2$-*c*-$C_6H_{11}$, $CH(CH_3)C_6H_5$ ou $CH(CH_3)$-*c*-$C_6H_{11}$.

Les agents édulcorants de l'invention se caractérisent aussi en ce qu'ils peuvent être salifiés par des acides ou des bases inorganiques ou organiques physiologiquement acceptables. Avantageusement, ces composés sont salifiés sous forme d'hydrochlorure ou de sels de sodium, potassium, ammonium, calcium et magnésium.

En d'autres termes, l'invention consiste à avoir selectionné comme groupe $R_0$ des dérivés des groupes parents hydrocarbonés suivants :

– 1,3-cycopentadièn-1-yle :

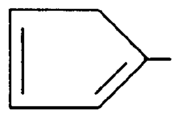

– 2,4-cyclopentadièn-1-yle :

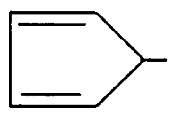

– phényle :

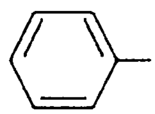

– indan-5-yle

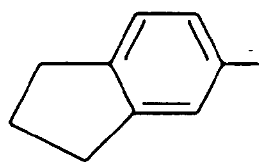

– indèn-2-yle :

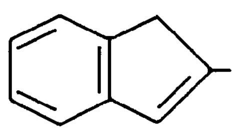

– indèn-5-yle :

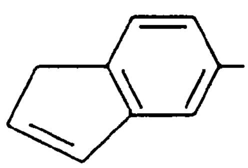 (1*H*-indèn-5-yle)

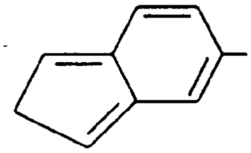 (2*H*-indèn-5-yle)

– indèn-6-yle :

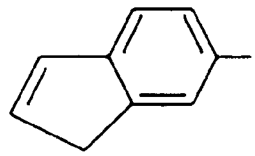

– 2-naphtyle :

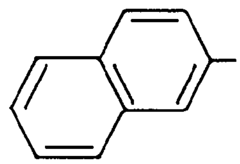

– 5,6,7,8-tétrahydro-2-naphtyle

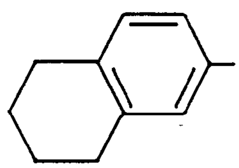

L'invention concerne également le procédé qui consiste à édulcorer les aliments, boissons, confiseries, patisseries, chewing gum, produits d'hygiène, cosmétiques, articles de toilette, produits pharmaceutiques et vétérinaires, et leurs équivalents, en leur ajoutant une quantité adéquate d'un ou plusieurs agents édulcorants selon la présente invention. Par "quantité adéquate", on désigne une quantité d'agent édulcorant suffisante pour produire la perception d'une saveur sucrée.

L'invention concerne aussi les préparations édulcorées suivant le procédé de la présente invention.

L'invention concerne également les compositions édulcorantes caractérisées en ce qu'elles comprennent une quantité adéquate d'au moins un agent édulcorant selon la présente invention, et un support ou agent de charge approprié.

L'invention concerne aussi les compositions édulcorantes caractérisées en ce qu'elles comprennent une quantité adéquate d'un ou plusieurs agents édulcorants selon la présente invention, et un ou plusieurs autres agents édulcorants.

Les composés de la présente invention se distinguent, pour la plupart, des composés décrits dans les demandes de brevet européen EP-A-O 195 730 ou 0 241 395 par leur plus grande stabilité en solution, et, pour les dérivés hétérocycliques azotés, par une meilleure solubilité et une plus grande rapidité de dissolution dans les milieux usuels (boissons gazeuses ...).

De plus, la présente invention permet d'accèder à des agents édulcorants qui sont parmi les plus puissants obtenus jusqu'alors, puisqu'on a pu obtenir un agent édulcorant qui est 130 000 fois plus sucré que le saccharose, sans commune mesure donc avec le pouvoir sucrant des principaux agents édulcorants actuellement utilisés, proposés, développés ou en cours d'expérimentation, et qui sont rappelés dans le tableau I ci-après.

Le pouvoir édulcorant des composés de la présente invention est donc souvent très fortement augmenté par rapport aux composés précédemment décrits, ce qui est totalement inattendu si l'on considère que toute modification, même légère, de la structure moléculaire d'un agent édulcorant peut entraîner une suppression de l'activité édulcorante, et ce d'autant que les relations entre la structure et l'activité édulcorante sont imprévisibles (voir par exemple M.G.J. BEETS, Structure-Activity Relationships in Human Chemoreception, Applied

Science Pub., London, 1978, pp. 259-362).

TABLEAU I

| Agents édulcorants | Pouvoir sucrant (saccharose = 1) |
|---|---|
| Xylitol | 1 |
| Cyclamate de sodium | 30 |
| Glycyrrhizine | 50 |
| Acésulfame-K | 200 |
| Aspartame | 200 |
| Stévioside | 300 |
| Saccharine | 400 |
| Néohespéridine dihydrochalcone | 1000 |
| Trichlorogalactosucrose | 2000 |
| Alitame | 2000 |
| Thaumatine | 2000 |
| Monelline | 2000 |

Un autre avantage très important des agents édulcorants de la présente invention est de donner souvent une saveur sucrée très pure, pratiquement identique à celle du saccharose, sans arrière-goût de réglisse (comme c'est le cas par exemple pour la glycyrrhizine ou la thaumatine) et sans arrière-goût métallique ou amer (comme c'est le cas par exemple pour la saccharine ou l'acésulfame-K).

Les autres avantages que l'on peut enfin attendre des agents édulcorants de la présente invention sont directement liés à leur activité édulcorante élevée, à savoir notamment leur faible prix de revient et leur sécurité d'utilisation due aux très faibles concentrations nécessaires pour obtenir une saveur sucrée.

Les composés de la présente invention peuvent être préparés par des méthodes variées déjà décrites dans la littérature (J. Med. Chem., 1978, 21, 773-781 ; Chem. Ber., 1966, 99, 1252-1257 ; Brit. Pat. No. 1587 258 ; J. Org. Chem., 1970, 35, 2067-2069 ; J. Org. Chem., 1986, 51, 1882-1884 ; Chem. Ber., 1967, 100, 591-604 ; J. Für. Prakt. Chemie, 1977, 319, 149-157 ; The Chemistry of Amidines and Imidates, S. Patai ed., Wiley-Interscience, 1975, p. 283-348).

Le procédé pour la préparation de tels agents édulcorants consiste à condenser un dérivé thiouréido ou thioamido de formule générale :

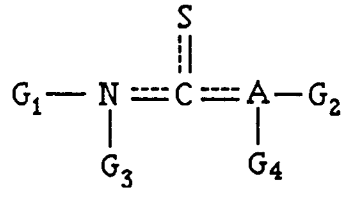

avec un composé de formule générale :

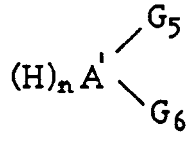

dans lesquelles :

. $G_1$ est $R_0$ ou $HOOCCHR_4$,

. $G_2$ est $R_2$ ou $HOOCCHR_4$,

. $G_3$, $G_4$ et $G_5$ sont H, $R_1$ ou $R_3$,

. $G_6$ est $R_0$, $R_2$ ou $HOOCCHR_4$,

. A' est N ou C,

. n est égal à 1 quand A' est N, et est égal à 2 quand A' est C ;

et dans lesquelles :

. $G_1$, $G_2$ et $G_6$ ne sont pas simultanément identiques,

. A et A' ne sont pas simultanément un atome de carbone,

. A' est N et $G_3$, $G_4$ et $G_5$ sont H lorsque $G_1$ ou $G_2$ ou $G_6$ sont $HOOCCHR_4$ ;

et dans lesquelles $R_0$, $R_1$, $R_2$, $R_3$, $R_4$ et A correspondent aux définitions données précédemment.

Les dérivés thiouréido peuvent être avantageusement obtenus par réaction d'un isothiocyanate sur une amine suivant l'une des réactions :

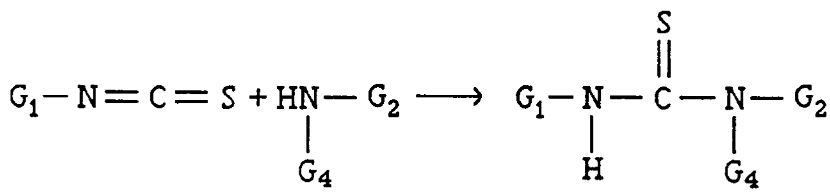

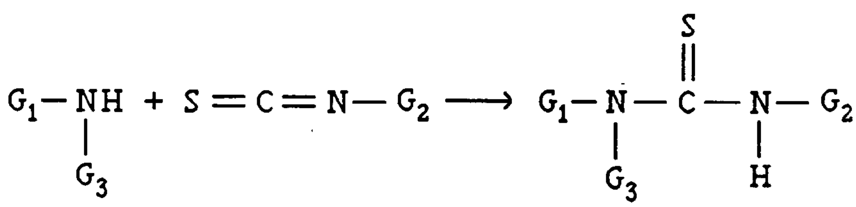

La condensation des dérivés thiouréido ou thioamido peut être avantageusement réalisée par activation de l'atome de soufre en le transformant en un groupe S-alkyle ou $SO_3H$, ou en le remplaçant par un groupe O-alkyl, O-$SO_2$aryl ou un atome d'halogène. En pratique, le dérivé thiouréido ou thioamido est avantageusement activé en le transformant en un dérivé S-méthylisothiouréido ou S-méthylisothioamido de formule générale :

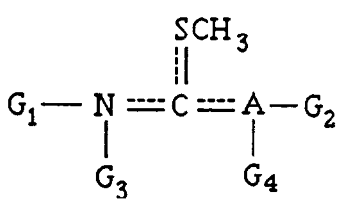

ceci par action d'un agent alkylant choisi dans le groupe comprenant l'iodure de méthyle et le diméthyl sulfate.

Le dérivé S-méthylisothiouréido préféré correspond à la formule générale :

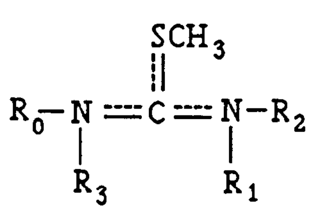

Lorsque A est N et lorsque $G_3$ et $G_4$ sont un atome d'hydrogène, la condensation peut être avantageusement réalisée en activant le dérivé thiouréido par transformation en un intermédiaire carbodiimide :

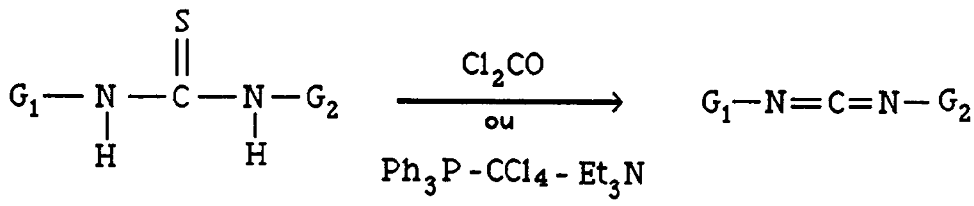


soit par traitement par le phosgène, soit par action d'un mélange équimoléculaire de $(C_6H_5)_3P$ - $CCl_4$ - $(C_2H_5)_3N$.

Enfin, lorsque A et A' sont N et que $G_3$ ou $G_4$ ou $G_5$ est H, la condensation du dérivé thiouréido avec l'amine peut avantageusement être réalisée par action de dicyclohexylcarbodiimide (DCC) suivant la réaction :

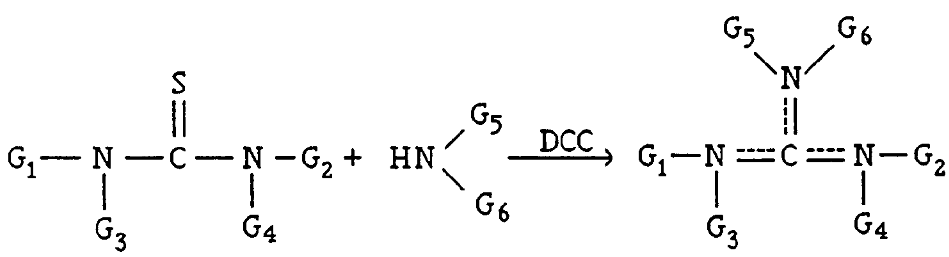


Avantageusement, en pratique :

L'une des méthodes de synthèse préférées pour obtenir les dérivés thiouréido consiste à faire réagir un isothiocyanate d'alkyle ou d'aryle avec une amine convenablement choisie. La réaction est conduite à température ambiante ou à ébullition suivant la réactivité des deux composés mis en présence, et elle est effectuée dans un solvant organique tel que l'éthanol, le méthanol, le chloroforme ou l'acétone. On peut ainsi réaliser les réactions suivantes :

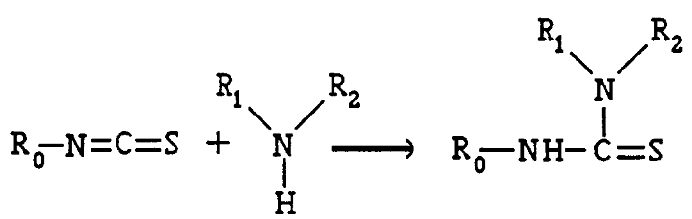

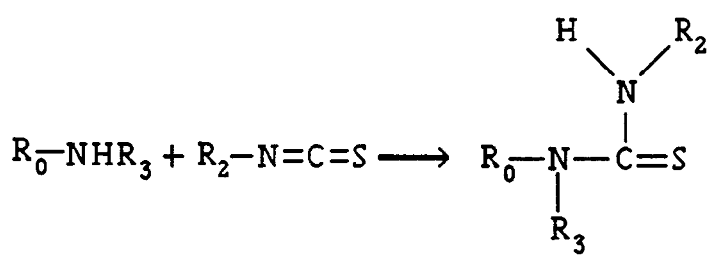

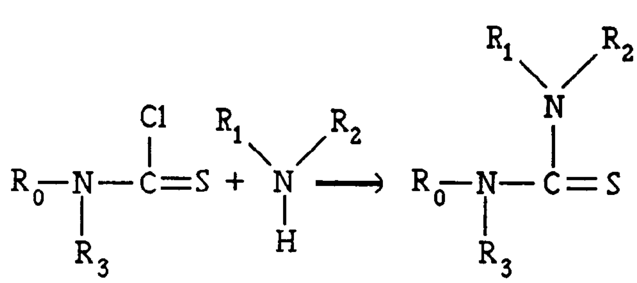

La condensation des dérivés thiouréido peut alors être avantageusement réalisée soit en remplaçant l'atome de soufre par un ligand activé (désigné par la lettre L dans les formules ci-dessous), soit en transformant

le dérivé thiouréido en un intermédiaire carbodiimide.

Dans la première méthode d'activation, le ligand activé est choisi de préférence dans l'ensemble constitué par les groupes S-alkyl, $SO_3H$, O-alkyl, $OSO_2$aryl, et les halogènes. La condensation consiste alors à faire réagir l'intermédiaire ainsi activé avec une amine convenablement choisie, en mettant en contact :

(1) 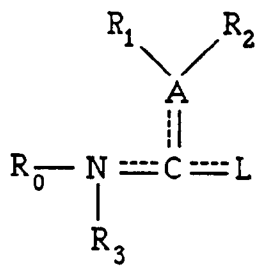 et $H_2N—CHR_4—COOH$ ,

(2) $R_0—NHR_3$ et 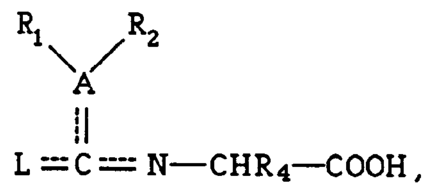

(3) 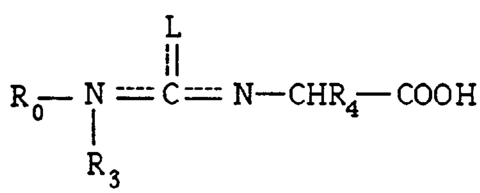 et 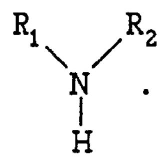 .

Dans une méthode de réalisation particulièrement avantageuse, le ligand activé L préféré est un groupe S-alkyle, de préférence un groupe S-$CH_3$, comme par exemple dans le dérivé S-méthylisothiouréido suivant :

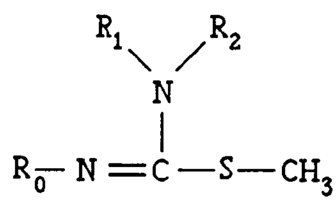

L'intermédiaire activé est avantageusement obtenu en traitant le dérivé thiouréido par un agent alkylant (iodure de méthyle, diméthyl sulfate) en solution dans un solvant organique comme l'acétone ou la 2-butanone, à une température comprise entre la température ambiante et l'ébullition. Le dérivé S-méthylisothiouréido est obtenu sous forme de sel (iodure, sulfate). Le sel est traité par une solution d'hydroxyde de sodium ou d'hydroxyde de potassium pour libérer la forme basique. La condensation avec l'α-amino acide est alors effectuée dans un mélange éthanol-eau, en présence d'une base telle que l'hydroxyde de sodium, l'hydroxyde de potassium ou une amine tertiaire (comme par exemple la triéthylamine), à des températures comprises entre la température ambiante et l'ébullition.

Dans la seconde méthode d'activation, l'intermédiaire carbodiimide est avantageusement obtenu par action du phosgène, ou par action d'un mélange équimoléculaire triphénylphosphine- tétrachlorure de carbone-amine tertiaire (triéthylamine par exemple) en solution dans un solvant organique tel que le tétrachlorure de carbone ou le dichlorométhane, à des températures comprises entre 0 °C et l'ébullition. La condensation consiste alors à faire réagir l'intermédiaire carbodiimide avec une amine convenablement choisie, en mettant en contact :

(4) $R_0-N=C=N-CHR_4-COOH$ et 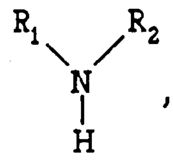 ,

(5) $R_0-N=C=N-R_2$ et $H_2N-CHR_4-COOH$ ,

(6) $R_0-NHR_3$ et $R_2-N=C=N-CHR_4-COOH$ .

Ces condensations peuvent être effectuées dans l'eau ou dans des solvants organiques tels que l'éthanol, le méthanol, l'acétone, le chloroforme, le tétrachlorure de carbone ou la pyridine, à une température pouvant varier de la température ambiante jusqu'à l'ébullition.

Dans certaines réactions (réactions 2 à 6), il peut être avantageux de protéger au préalable le groupe carboxyle de l'α-amino acide, sous forme par exemple d'un ester (ester méthylique, éthylique, *tert*-butylique, benzylique). Il est alors nécessaire, pour obtenir les composés de l'invention, d'éliminer le groupe protecteur par le moyen le plus approprié qui peut être, par exemple, une saponification par une solution d'hydroxyde de sodium ou une hydrolyse par une solution de chlorure d'hydrogène.

Les composés de l'invention peuvent exister, suivant la nature de $R_0$, $R_1$, $R_2$, $R_3$ et A, sous forme de zwitterion ou sous forme acide. Ils peuvent donc être salifiés par des acides ou des bases inorganiques ou organiques physiologiquement acceptables. L'une des méthodes de choix pour préparer ces sels consiste à concentrer à sec sous vide un mélange, en solution aqueuse, d'un composé de l'invention et d'un équivalent d'un acide ou d'une base inorganique ou organique. Les sels préférés de l'invention sont les hydrochlorures et les sels de sodium, potassium, ammonium, calcium et magnésium.

La purification des composés de l'invention a été réalisée selon les techniques stantards telles que la recristallisation ou la chromatographie. Leur structure et leur pureté a été contrôlée par les techniques classiques (chromatographie sur couche mince, chromatographie liquide haute performance, spectrométrie infra-rouge, résonance magnétique nucléaire, analyse élémentaire).

Les présents agents édulcorants peuvent exister comme un mélange en équilibre de formes tautomères. C'est ainsi par exemple, quand A est N, et $R_1$ et $R_3$ sont H, on a :

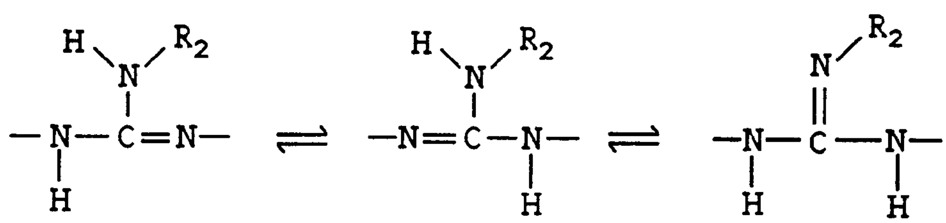

Quand A est N, $R_1$ est $CH_3$, et $R_3$ est H, on a:

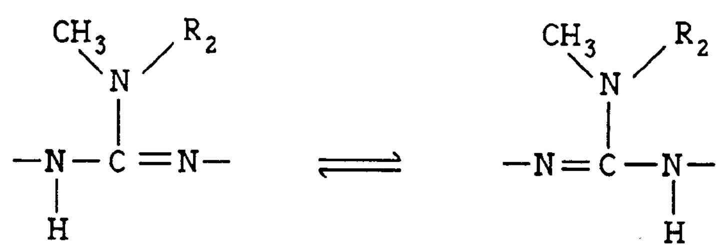

Quand A est N, $R_1$ est H, et $R_3$ est $CH_3$, on a:

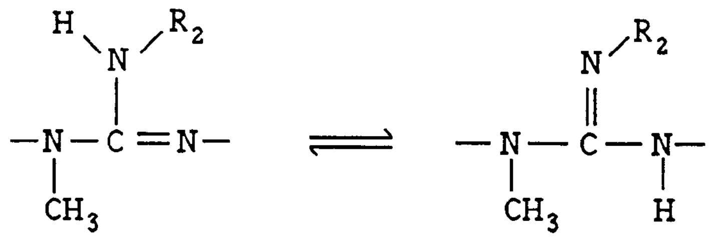

Quand A est C, et $R_3$ est H, on a:

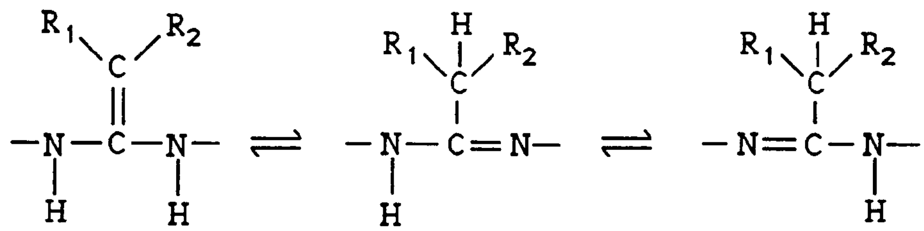

C'est la raison pour laquelle les agents édulcorants de la présente invention sont représentés, dans la formule générale, par un hybride de résonance , et, dans la partie descriptive, par une de leurs formes tautomères, tout en sachant que la forme tautomère figurée est immanquablement en équilibre avec les autres formes tautomères, les proportions respectives des tautomères variant suivant la nature des substituants $R_0$, $R_1$, $R_2$ et $R_3$, et suivant le pH.

Les agents édulcorants de la présente invention ont pour utilité de pouvoir être ajoutés à tout produit comestible dans lequel on désire apporter un goût sucré, à condition qu'on les ajoute en proportions suffisantes pour atteindre le niveau d'édulcoration désiré. La concentration optimale d'utilisation de l'agent édulcorant dépendra de facteurs divers tels que, par exemple, le pouvoir sucrant de l'agent édulcorant, les conditions de stockage et d'utilisation des produits, les constituants particuliers des produits, le profil gustatif des produits comestibles et le niveau d'édulcoration désiré. Toute personne du métier peut facilement déterminer la proportion optimale d'agent édulcorant qui doit être employée pour l'obtention d'un produit comestible en réalisant des analyses sensorielles de routine. Les agents édulcorants de la présente invention sont, en général, ajoutés aux produits comestibles dans des proportions d'environ 0,0001 à environ 0,2 pour cent en poids du produit comestible, avantageusement d'environ 0,0005 à environ 0,15 pour cent en poids, et préférentiellement d'environ 0,001 à environ 0,1 pour cent en poids. Les produits concentrés contiendront évidemment des pourcentages plus élevés d'agent(s) édulcorant(s), et seront ensuite dilués suivant les intentions finales d'utilisation.

Les produits susceptibles d'être édulcorés par les agents édulcorants de la présente invention comprennent tous les produits pour lesquels on désire un composant de goût sucré, notamment les produits alimentaires (pour la consommation humaine ou animale), les boissons (boissons alcooliques, boissons non alcooliques, jus, boissons gazeuses), les confiseries, les pâtisseries, le chewing gum, les produits d'hygiène, les cosmétiques, les produits pharmaceutiques et vétérinaires.

Les agents édulcorants de la présente invention peuvent être ajoutés sous forme pure aux produits comestibles pour leur communiquer un goût sucré. Toutefois, par suite du pouvoir sucrant élevé des présents agents édulcorants, ils sont généralement mélangés à un support ("carrier") ou à un agent de charge approprié ("bulking agent"). Avantageusement, les supports ou agents de charge appropriés sont choisis dans le groupe constitué par le polydextrose, l'amidon, les maltodextrines, la cellulose, la méthylcellulose, la carboxyméthylcellulose et autres dérivés de la cellulose, l'alginate de sodium, les pectines, les gommes, le lactose, le maltose, le glucose, la leucine, le glycérol, le mannitol, le sorbitol, le bicarbonate de sodium, les acides phosphorique, citrique, tartrique, fumarique, benzoïque, sorbique, propionique, et leurs sels de sodium, potassium et calcium.

Les présents agents édulcorants peuvent, dans un produit comestible, être employés seuls, comme unique agent édulcorant, ou sous forme de mélanges de deux ou plusieurs agents édulcorants de la présente invention. Les présents agents édulcorants peuvent, en outre, être utilisés en combinaison avec d'autres agents édulcorants tels que les sucres (saccharose), le sirop de maïs, le fructose, les dérivés dipeptidiques sucrés (aspartame, alitame), la néohespéridine dihydrochalcone, l'isomaltulose hydrogéné, le stévioside, les sucres L, la glycyrrhizine, le xylitol, le sorbitol, le mannitol, l'acésulfame-K, la saccharine et ses sels de sodium, potassium, ammonium et calcium, l'acide cyclamique et ses sels de sodium, potassium et calcium, le trichlorogalactosucrose, la monelline, la thaumatine.

Le pouvoir édulcorant des composés préparés dans les exemples suivants a été évalué par un groupe de huit goûteurs expérimentés. Pour cela, les composés, en solution aqueuse à des concentrations variables, sont comparés, sur le plan gustatif, à une solution témoin de saccharose à 2 % et dans certains cas à 5 % et 10 %, c'est-à-dire à des concentrations correspondant à celles utilisées lors d'un usage courant. Le pouvoir sucrant des édulcorants de synthèse varie en effet suivant la concentration de la solution de saccharose utilisée comme référence. Le pouvoir édulcorant du composé testé par rapport au saccharose correspond alors au rapport pondéral qui existe entre le composé et le saccharose à égale intensité édulcorante, c'est-à-dire quand les saveurs sucrées de la solution du composé testé et de la solution témoin de saccharose sont considérées, par une majorité de goûteurs, avoir la même intensité édulcorante.

La manière dont l'invention peut être réalisée et les avantages qui en découlent ressortiront mieux des exemples de réalisation qui suivent.

EXEMPLE 1 :

Synthèse de l'acide N-[N-cyclooctylamino(1-pyrrolylimino)méthyl]-2-aminoéthanoïque :

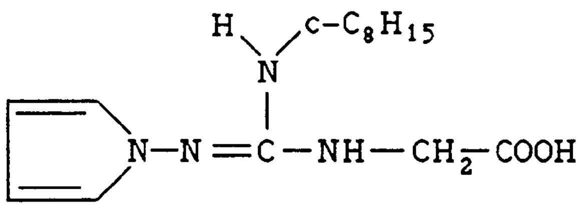

Etape 1 : Préparation de la N-cyclooctyl-N'-(1-pyrrolyl) thiourée :

Une solution de 1 g (12,1 mmol) de 1-amino pyrrole et de 2,06 g (12,1 mmol) de cyclooctyl isothiocyanate dans 30 $cm^3$ d'acétonitrile est chauffée durant 30 h à ébullition.

Après concentration à sec sous vide, le résidu obtenu est dissous dans 30 $cm^3$ d'éther éthylique puis est lavé par une solution d'acide chlorhydrique 1 N (3 × 10 $cm^3$). La solution éthérée est séchée sur sulfate de sodium anhydre puis concentrée à sec sous vide. On obtient 2,2 g d'une huile qui est purifiée par chromatographie sur colonne ( gel de silice 60 ; éluant : chloroforme) ; on obtient finalement 0,75 g (rendement 25 %) d'un solide dont le point de fusion est de 125 °C.

Etape 2 : Préparation de l'acide N-[N-cyclooctylamino(1-pyrrolylimino)méthyl]-2-aminoéthanoïque sous forme d'hydrochlorure :

Une suspension de 0,75 g (2,99 mmol) de la thiourée précédemment obtenue, de 0,52 g (4 mmol) de glycine *tert*-butyl ester et de 0,82 g (4 mmol) de dicyclohexylcarbodiimide dans 30 $cm^3$ d'acétate d'éthyle est chauffée durant 15 h à 75 °C. La solution est concentrée à sec sous vide et le résidu est dissous dans 50 $cm^3$ d'éther éthylique. On extrait la solution éthérée obtenue par une solution d'acide chlorhydrique 0,25 N (4 × 50 $cm^3$). Cette solution aqueuse est ensuite lavée par l'éther éthylique (2 × 30 $cm^3$) puis portée à pH ≈ 10 par addition d'une solution d'hydroxyde de sodium 1 N. Le précipité huileux formé est extrait par l'éther éthylique (5 × 30 $cm^3$). La solution dans l'éther éthylique est séchée sur sulfate de sodium anhydre puis est concentrée à sec. On obtient 0,75 g (rendement 69 %) de *tert*-butyl N-[N-cyclooctylamino(1-pyrrolimino)méthyl]-2-aminoéthanoate sous forme d'une huile qui cristallise lentement (point de fusion 102 °C).

A 2,8 $cm^3$ d'une solution de HCl 7,5 N dans le dioxane est ajouté 0,75 g du composé précédemment obtenu. La solution est agitée durant 2 h à O °C, puis 30 minutes à 20 °C, et concentrée sous vide. Le résidu huileux obtenu est trituré dans l'éther éthylique anhydre pendant plusieurs heures, ce qui permet d'obtenir 0,5 g d'un produit amorphe (rendement 71 % ; point de fusion, 100 °C).

Le pouvoir édulcorant de ce composé correspond approximativement, sur une base pondérale, à 1 000 (mille) fois celui du saccharose (par comparaison avec une solution de saccharose à 2 %).

EXEMPLE 2 :

Synthèse de l'acide N-[N-cyclooctylamino(2-thiazolylimino)méthyl]-2-aminoéthanoïque :

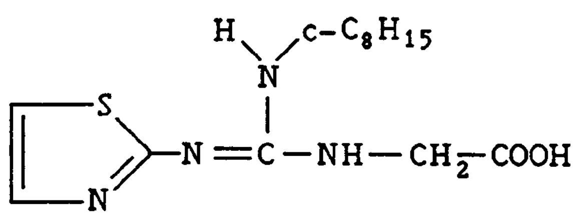

Ce composé est obtenu à partir de la N-cyclooctyl-N'-(2-thiazolyl) thiourée et de la glycine *tert*-butyl ester, en suivant le protocole expérimental décrit dans l'exemple 1 (rendement 90 % ; point de fusion 140 °C).

Le pouvoir édulcorant de ce composé correspond approximativement, sur une base pondérale, à 150 (cent cinquante) fois celui du saccharose (par comparaison avec une solution de saccharose à 2 %).

EXEMPLE 3 :

Synthèse de l'acide N-[N-(S)-α-méthylbenzylamino (2-pyridylimino)méthyl]-2-aminoéthanoïque :

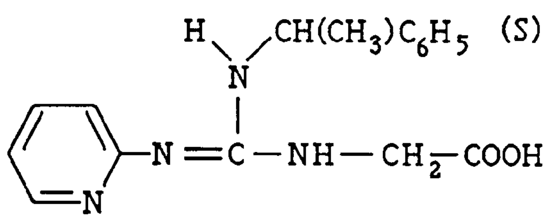

Ce composé est obtenu à partir de la glycine et de la N-(S)-α-méthylbenzyl-N'-(2-pyridyl)-S-méthylisothiourée, en suivant le protocole expérimental décrit dans l'exemple 9 (rendement 28 % ; point de fusion 160 °C).

Le pouvoir édulcorant de ce composé est, sur une base pondérale, inférieur à 10 (dix) fois celui du saccharose (par comparaison avec une solution de saccharose à 2 %) avec un arrière-goût amer.

EXEMPLE 4 :

Synthèse de l'acide N-[N-benzylamino(3-pyridylimino)méthy]-2-aminoéthanoïque :

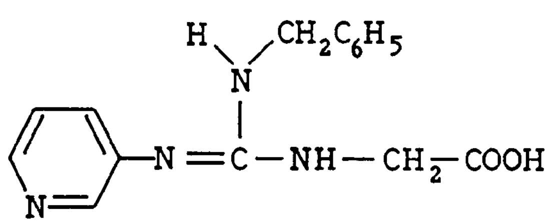

Etape 1 : Préparation de la N-benzyl-N'-(3-pyridyl) thiourée :

4,41 g (46,8 mmol) de 3-aminopyridine et 7 g (46,8 mmol) de benzyl isothiocyanate sont mélangés dans 50 $cm^3$ d'éthanol à 95 %. Après 4 heures d'agitation à 20 °C, on ajoute 300 $cm^3$ d'éther éthylique. Après filtration et lavage par l'éther éthylique du précipité formé, 10,4 g (rendement 91 %) du dérivé thiouréido (point de fusion 137 °C) sont obtenus.

Etape 2 : Préparation de la N-benzyl-N'-(3-pyridyl)carbodiimide :

4,86 g (20 mmol) de la thiourée précédemment obtenue, en solution dans le tétrahydrofurane (THF) anhydre (30 $cm^3$), sont ajoutés, goutte à goutte, sous agitation et à une température de 0 °C, à 19 $cm^3$ d'une solution à 20 % de phosgène dans le toluène. Après 5 heures à 0 °C le mélange est évaporé sous vide. Le résidu repris

par du THF (30 $cm^3$) et de la diisopropyléthylamine (6,8 $cm^3$) à 0 °C, est évaporé à sec puis extrait par de l'hexane (3 × 50 $cm^3$). Les extraits conduisent, après évaporation, à la carbodiimide qui est récupérée sous forme d'une huile (3,84 g ; rendement 91 %).

Etape 3 : Préparation de l'acide N-[N-benzylamino(3-pyridylimino)méthyl]-2-aminoéthanoïque :

Une solution dans la pyridine (10 $cm^3$) de 1 g (4,7 mmol) de la carbodiimide précédemment obtenue et de 0,85 g (9,5 mmol) de glycine méthyl ester est agitée durant 2 heures à 20 °C. Le mélange, traité par l'eau (100 $cm^3$) et par une solution de $NaHCO_3$ à 5 % (10 $cm^3$), est extrait par l'éther éthylique (3 × 50 $cm^3$). Les extraits sont séchés sur sulfate de sodium et concentrés à sec pour donner 0,78 g d'une huile qui cristallise rapidement. Le solide, traité par l'éther éthylique et filtré (0,42 g ; point de fusion 156 °C), est mis en solution dans un mélange d'une solution 1N d'hydroxyde de sodium (1,4 $cm^3$) et de méthanol (3 $cm^3$). Après 12 heures à 35 °C, le mélange est concentré à sec, repris dans 10 $cm^3$ d'eau et la solution ainsi obtenue est lavée par le dichlorométhane (3 × 15 $cm^3$).

Après neutralisation à pH 7 par une solution d'HCl 1 N et concentration de la solution à sec, le résidu obtenu est extrait par l'acétone (2 × 50 $cm^3$) à ébullition.

Après concentration à sec, 0,24 g (rendement 17%) du produit désiré est obtenu (point de fusion 146 °C).

Le pouvoir édulcorant de ce composé correspond approximativement, sur une base pondérale, à 4 000 (quatre mille) fois celui du saccharose (par comparaison avec une solution de saccharose à 2 %).

EXEMPLE 5 :

Synthèse de l'acide N-[N-(S)-α-méthylbenzylamino(4,6-diméthyl-2-pyrimidinylimino)méthyl]-2-aminoéthanoïque:

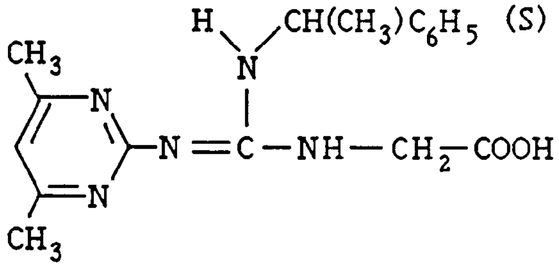

Ce composé est obtenu à partir de la glycine et de la N-(S)-α-méthylbenzyl-N'-(4,6-diméthyl-2-pyrimidinyl)-S-méthylisothiourée, en suivant le protocole expérimental décrit dans l'exemple 9 (rendement 8 % ; point de fusion 150 °C).

Le pouvoir édulcorant de ce composé est, sur une base pondérale, inférieur à 10 (dix) fois celui du saccharose (par comparaison avec une solution de saccharose à 2 %) avec un arrière-goût amer.

EXEMPLE 6 :

Synthèse de l'acide N-[N-(S)-α-méthylbenzylamino (5-indanylimino)méthyl]-2-aminoéthanoïque :

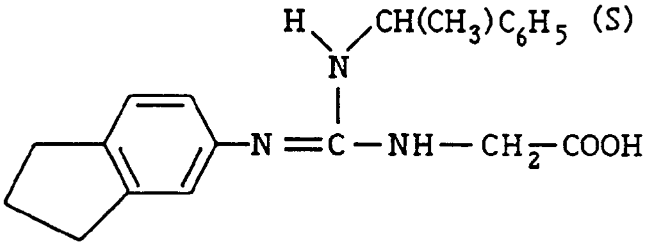

Ce composé est obtenu à partir de la glycine et de la N-(S)-α-méthylbenzyl-N'-(5-indanyl)-S-méthyl isothiourée, en suivant le protocole expérimental décrit dans l'exemple 9 (rendement 18 % ; point de fusion 165 °C).

Le pouvoir édulcorant de ce composé correspond approximativement, sur une base pondérale, à 2 000 (deux mille) fois celui du saccharose (par comparaison avec une solution de saccharose à 2 %).

EXEMPLE 7 :

Synthèse de l'acide N-[N-(S)-α-méthylbenzylamino (1*H*-indazol-5-ylimino)méthyl]-2-aminoéthanoïque :

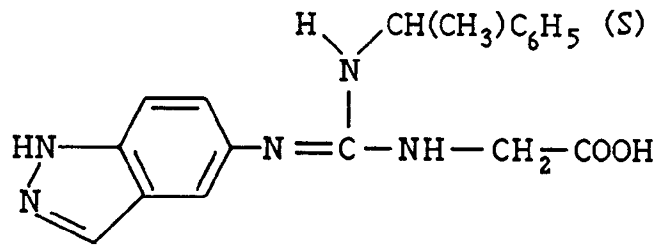

Ce composé est obtenu à partir de la glycine et de la N-(S)-α-méthylbenzyl-N'-(1H-indazol-5-yl)-S-méthyl isothiourée, en suivant le protocole expérimental décrit dans l'exemple 9 (rendement 54 %, point de fusion 176 °C).

Le pouvoir édulcorant de ce composé correspond approximativement, sur une base pondérale, à 1 100 (mille cent) fois celui du saccharose (par comparaison avec une solution de saccharose à 2 %).

EXEMPLE 8 :

Synthèse de l'acide N-[N-(S)-α-méthylbenzylamino (1*H*-indazol-6-ylimino)méthyl]-2-aminoéthanoïque :

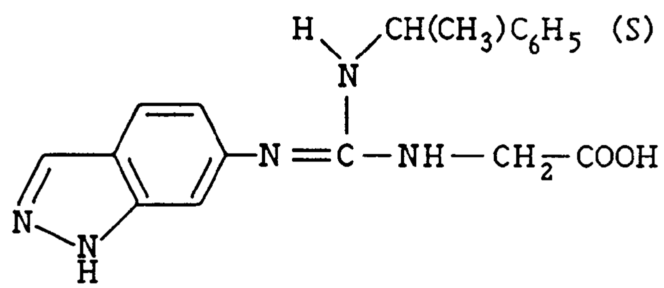

Ce composé est obtenu à partir de la glycine et de la N-(S)-α-méthylbenzyl-N'-(1*H*-indazol-6-yl)-S-méthylisothiourée, en suivant le protocole expérimental décrit dans l'exemple 9 (rendement 43 %, point de fusion 188 °C).

Le pouvoir édulcorant de ce composé correspond approximativement, sur une base pondérale, à 24 000 (vingt quatre mille) fois celui du saccharose (par comparaison avec une solution de saccharose à 2 %).

EXEMPLE 9 :

Synthèse de l'acide N-[N-cyclooctylamino(1*H*-indazol-6-ylimino)méthyl]-2-aminoéthanoïque :

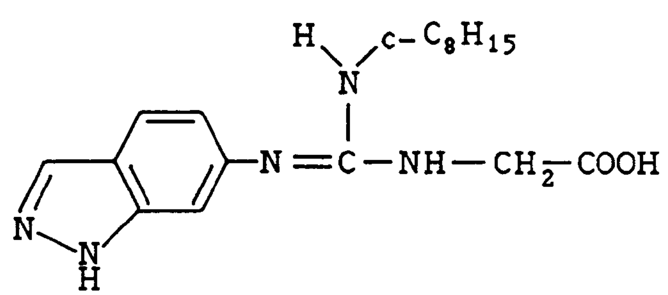

Etape 1 : Préparation de la N-cyclooctyl-N'-(1*H*-indazol-6-yl) thiourée :

4,81 g (37,8 mmol) de cyclooctylamine et 6 g (34,2 mmol) de 1*H*-indazol-6-yl isothiocyanate sont mélangés dans 50 $cm^3$ d'éthanol à 95 %. Après 4 heures d'agitation à 20 °C, la solution est concentrée à sec sous vide. Le résidu est dissous dans 20 $cm^3$ d'acétone et la solution est traitée par addition de 300 $cm^3$ d'éther éthylique. Après filtration et lavage par l'éther éthylique du précipité ainsi formé, 2,6 g (rendement 54 %) du dérivé thiouréido (point de fusion 160 °C) sont obtenus.

Etape 2 : Synthèse de la N-cyclooctyl-N'-(1*H*-indazol-6-yl)-S-méthylisothiourée :

Un mélange de 2,6 g (8,6 mmol) du composé précédemment obtenu et de 1,83 g (12,9 mmol) d'iodure de méthyle est agité dans 20 $cm^3$ de 2-butanone à 20 °C pendant 48 heures. Après filtration du précipité formé et lavage par l'éther éthylique, 2,2 g (rendement 58 %, point de fusion 198 °C) ) du dérivé S-méthylisothiouréido sont obtenus sous forme d'hydroiodure.

Ce sel est alors dissous dans 40 $cm^3$ d'une solution 1N d'hydroxyde de sodium. Le mélange alcalin résultant est extrait par du dichlorométhane (3 × 50 $cm^3$). Après séchage sur du sulfate de sodium anhydre et concentration à sec, 1,4 g (rendement 89 %, point de fusion 132 °C) du dérivé isothiouréido est obtenu.

Etape 3 : Préparation de l'acide N-[N-cyclooctylamino(1*H*-indazol-6-ylimino)méthyl]-2-aminoéthanoïque :

Un mélange de 0,5 g (6,6 mmol) de glycine et de 0,26 g (6,6 mmol) d'hydroxyde de sodium dans 5 $cm^3$ d'eau est ajouté à une solution de 1,4 g (4,4 mmol) de N-cyclooctyl-N'-(1*H*-indazol-6-yl)-S-méthylisothiourée dans 20 $cm^3$ d'éthanol à 95 %. Le mélange est chauffé à 70 °C durant 20 heures. Après refroidissement, la solution est concentrée à sec et le résidu est dissous dans 30 $cm^3$ d'eau. La solution résultante est lavée par du dichlorométhane (3 × 30 $cm^3$) puis acidifiée par une solution d'HCl 2N jusqu'à ce qu'un pH de 7 soit obtenu. Le dérivé guanidino précipite et est alors isolé par filtration. 0,78 g du produit (rendement 51 %, point de fusion 138 °C) est obtenu.

Le pouvoir édulcorant de ce composé correspond approximativement, sur une base pondérale, à 130 000 (cent trente mille) fois celui du saccharose (par comparaison avec une solution de saccharose à 2 %), à 100 000 (cent mille) fois par rapport à une solution à 5 %, et à 90 000 (quatre vingt dix mille) fois par rapport à une solution à 10 %.

<u>EXEMPLE 10</u> :

Synthèse de l'acide N-[N-(S)-α-méthylbenzylamino(3,4-méthylènedioxyphénylimino)méthyl]-2-aminoéthanoïque:

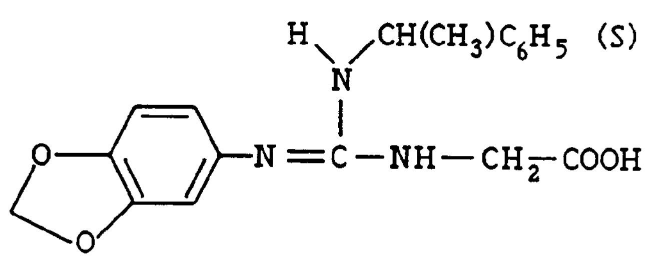

Ce composé est obtenu à partir de la glycine et de la N-(S)-α-méthylbenzyl-N'-(3,4-méthylènedioxyphényl)-S-méthylisothiourée, en suivant le protocole expérimental décrit dans l'exemple 9 (rendement 41 %, point de fusion 213 °C).

Le pouvoir édulcorant de ce composé correspond approximativement, sur une base pondérale, à 3 500 (trois mille cinq cent) fois celui du saccharose (par comparaison avec une solution de saccharose à 2 %).

EXEMPLE 11 :

Synthèse de l'acide N-[N-cyclooctyl(5-benzofurazanylimino)méthyl]-2-aminoéthanoïque :

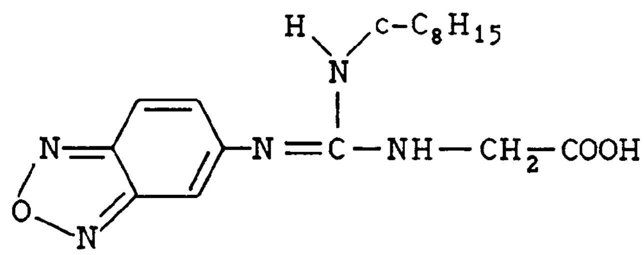

Ce composé est obtenu à partir du *tert*-butyl N-[N-cyclooctylamino(5-benzofurazanylimino)méthyl]-2-aminoéthanoate (obtenu en suivant le protocole expérimental décrit dans l'exemple 1) par traitement durant 1 heure par une solution d'acide trifluoroacétique suivi d'une trituration dans l'éther éthylique (rendement 26 % ; point de fusion 135 °C, sous forme de sel de l'acide trifluoroacétique).

Le pouvoir édulcorant de ce composé correspond approximativement, sur une base pondérale, à 40 000 (quarante mille) fois celui du saccharose (par comparaison avec une solution de saccharose à 2 %).

EXEMPLE 12 :

Synthèse de l'acide N-[N-(S)-α-méthylbenzylamino (2-naphtylimino)méthyl]-2-aminoéthanoïque :

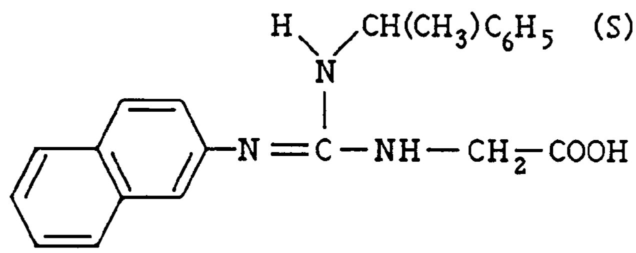

Ce composé est obtenu à partir de la glycine et de la N-(S)-α-méthylbenzyl-N'-(2-naphtyl)-S-méthylisothiourée, en suivant le protocole expérimental décrit dans l'exemple 9 (rendement 19 %, point de fusion 164 °C).

Le pouvoir édulcorant de ce composé correspond approximativement, sur une base pondérale, à 9 000 (neuf mille) fois celui du saccharose (par comparaison avec une solution de saccharose à 2 %).

EXEMPLE 13 :

Synthèse de l'acide N-[N-(S)-α-méthylbenzylamino(3-quinolylimino)méthyl]-2-aminoéthanoïque :

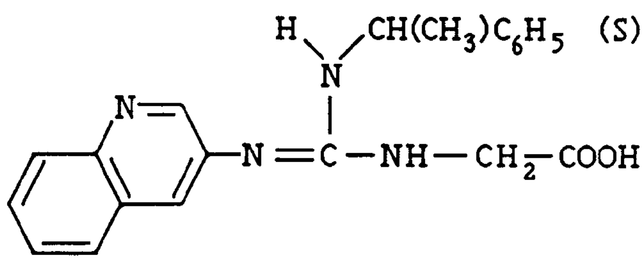

Ce composé est obtenu à partir de la glycine et de la N-(S)-α-méthylbenzyl-N'-(3-quinolyl)-S-méthylisothiourée, en suivant le protocole expérimental décrit dans l'exemple 9 (rendement 26 %, point de fusion 188 °C).

Le pouvoir édulcorant de ce composé correspond approximativement, sur une base pondérale, à 15 000 (quinze mille) fois celui du saccharose (par comparaison avec une solution de saccharose à 2 %).

EXEMPLE 14 :

Synthèse de l'acide N-[N-(S)-α-méthylbenzylamino(6-quinolylimino)méthyl]-2-aminoéthanoïque :

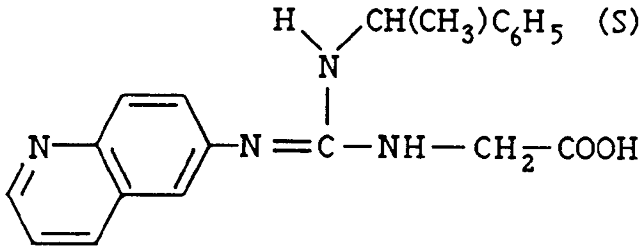

Ce composé est obtenu à partir de la glycine et de la N-(S)-α-méthylbenzyl-N'-(6-quinolyl)-S-méthylisothiourée, en suivant le protocole expérimental décrit dans l'exemple 9 (rendement 15 %, point de fusion 161 °C).

Le pouvoir édulcorant de ce composé correspond approximativement, sur une base pondérale, à 5 000 (cinq mille) fois celui du saccharose (par comparaison avec une solution de saccharose à 2 %).

EXEMPLE 15 :

Synthèse de l'acide N-[N-cyclooctyl(6-quinolylimino)méthyl]-2-aminoéthanoïque :

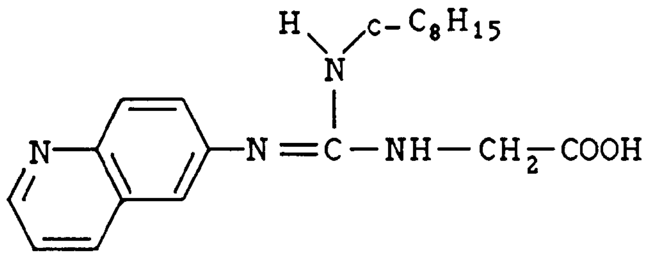

Ce composé est obtenu à partir de la glycine et de la N-cyclooctyl-N'-(6-quinolyl)-S-méthylisothiourée, en suivant le protocole expérimental décrit dans l'exemple 9 (rendement 20 %, point de fusion 150 °C).

Le pouvoir édulcorant de ce composé correspond approximativement, sur une base pondérale, à 10 000 (dix mille) fois celui du saccharose (par comparaison avec une solution de saccharose à 2 %).

EXEMPLE 16 :

Synthèse de l'acide N-[N-(S)-α-méthylbenzylamino(1,4-benzodioxan-6-ylimino)méthyl]-2-aminoéthanoïque :

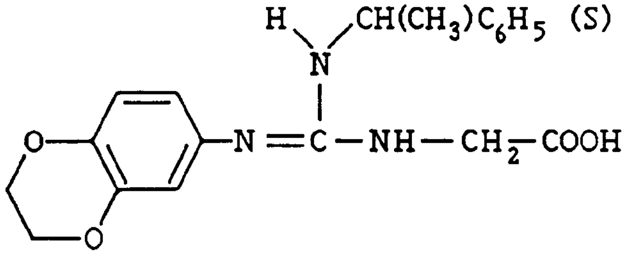

Ce composé est obtenu à partir de la glycine et de la N-(S)-α-méthylbenzyl-N'-(1,4-benzodioxan-6-yl)-S-méthylisothiourée, en suivant le protocole expérimental décrit dans l'exemple 9 (rendement 41 %, point de

fusion 205 °C).

Le pouvoir édulcorant de ce composé correspond approximativement, sur une base pondérale, à 700 (sept cents) fois celui du saccharose (par comparaison avec une solution de saccharose à 2 %).

Les pouvoirs édulcorants obtenus avec les différents composés cités dans les Exemples 1 à 16 sont récapitulés dans le Tableau II ci-après ; les pouvoirs édulcorants ainsi donnés ont été évalués, sur une base pondérale, par rapport à une solution de saccharose à 2 %.

TABLEAU II

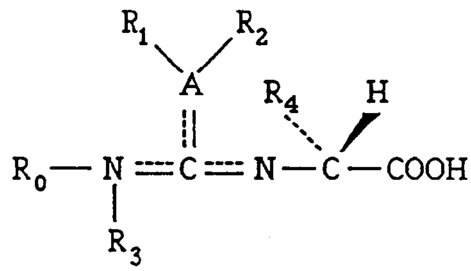

| Composé | $R_0$ | A | $R_1$ | $R_2$ | $R_3$ | $R_4$ | Pouvoir édulcorant |
|---|---|---|---|---|---|---|---|
| 1 | 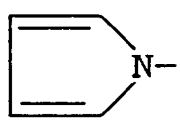 | N | H | $c$-$C_8H_{15}$ | - | H | 1 000 |
| 2 | 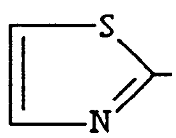 | N | H | $c$-$C_8H_{15}$ | - | H | 150 |
| 3 | 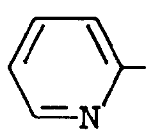 | N | H | (S)$CH(CH_3)C_6H_5$ | - | H | <10 |
| 4 | 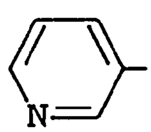 | N | H | $CH_2C_6H_5$ | - | H | 4 000 |
| 5 | 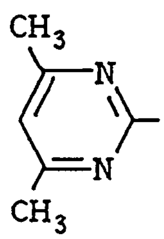 | N | H | (S)$CH(CH_3)C_6H_5$ | - | H | <10 |

| Composé | $R_0$ | A | $R_1$ | $R_2$ | $R_3$ | $R_4$ | Pouvoir édulcorant |
|---|---|---|---|---|---|---|---|
| 6 | 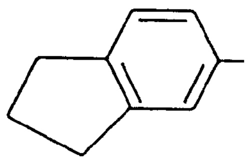 | N | H | $(S)CH(CH_3)C_6H_5$ | - | H | 2 000 |
| 7 | 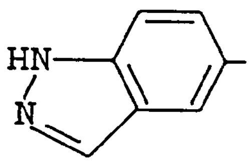 | N | H | $(S)CH(CH_3)C_6H_5$ | - | H | 1 100 |
| 8 | 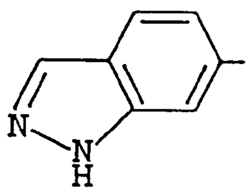 | N | H | $(S)CH(CH_3)C_6H_5$ | - | H | 24 000 |
| 9 | 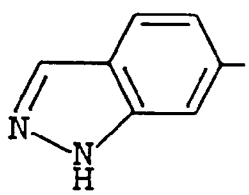 | N | H | $c-C_8H_{15}$ | - | H | 130 000 |
| 10 | 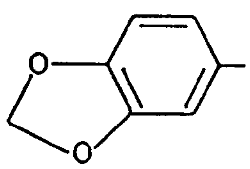 | N | H | $(S)CH(CH_3)C_6H_5$ | - | H | 3 500 |
| 11 | 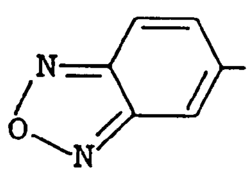 | N | H | $c-C_8H_{15}$ | - | H | 40 000 |

| Composé | $R_0$ | A | $R_1$ | $R_2$ | $R_3$ | $R_4$ | Pouvoir édulcorant |
|---|---|---|---|---|---|---|---|
| 12 | 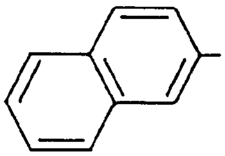 | N | H | $(S)CH(CH_3)C_6H_5$ | - | H | 9 000 |
| 13 | 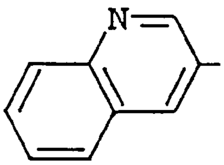 | N | H | $(S)CH(CH_3)C_6H_5$ | - | H | 15 000 |
| 14 | 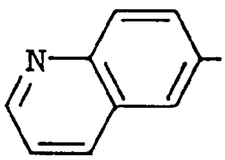 | N | H | $(S)CH(CH_3)C_6H_5$ | - | H | 5 000 |
| 15 | 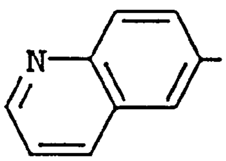 | N | H | $c$-$C_8H_{15}$ | - | H | 10 000 |
| 16 | 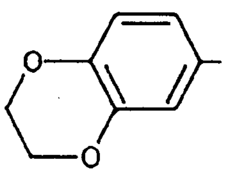 | N | H | $(S)CH(CH_3)C_6H_5$ | - | H | 700 |

## Revendications

**Revendications pour les Etats contractants suivants: AT, BE, CH, LI, DE, FR, GB, IT, LU, NL, SE**

1. Agents édulcorants de formule générale :

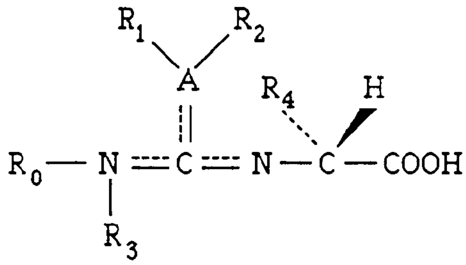

en incluant les formes tautomères et les sels physiologiquement acceptables,

– dans laquelle A est choisi dans le groupe comprenant N et C ;

– dans laquelle $R_1$ est un atome d'hydrogène ou un groupe hydrocarboné ou un groupe hydrocarboné modifié, ayant jusqu'à 4 atomes de carbone, saturé ou insaturé, acyclique, cyclique ou mixte, et dans lequel, dans le groupe hydrocarboné modifié :

. 1 à 2 atomes de carbone peuvent être remplacés par 1 à 2 hétéroatomes, identiques ou différents, choisis dans le groupe comprenant les atomes d'azote, oxygène, soufre, chlore, brome et iode,

. et 1 à 3 atomes d'hydrogène peuvent être remplacés par 1 à 3 atomes de fluor ;

– dans laquelle $R_2$ est un groupe hydrocarboné ou un groupe hydrocarboné modifié, de 2 à 13 atomes de carbone, saturé ou insaturé, acyclique, cyclique ou mixte, et dans lequel, dans le groupe hydrocarboné modifié :

. 1 à 4 atomes de carbone peuvent être remplacés par 1 à 4 hétéroatomes, identiques ou différents, choisis dans le groupe comprenant les atomes l'azote, oxygène, soufre, chlore, brome et iode,

. et 1 à 5 atomes d'hydrogène peuvent être remplacés par 1 à 5 atomes de fluor ;

– dans laquelle $R_1$ et $R_2$ peuvent être fusionnés ;

– dans laquelle $R_3$ est choisi dans le groupe comprenant un atome d'hydrogène ou un groupe alkyle $C_1$-$C_3$ ;

– dans laquelle $R_4$ est choisi dans le groupe comprenant :

H et

$CH_3$ ;

caractérisé en ce que $R_o$ est choisi dans le groupe comprenant :

. les groupes hétérocycliques dérivés des groupes parents hydrocarbonés 1,3-cyclopentadièn-1-yle, 2,4-cyclopentadièn-1-yle et phényle par remplacement de 1 à 3 atomes de carbone du cycle parent hydrocarboné par 1 à 3 atomes d'azote, d'oxygène et de soufre,

et dans lesquels les atomes d'hydrogène des positions 3 et 4 des groupes parents 1,3-cyclopentadièn-1-yle, 2,4-cyclopentadièn-1-yle et des positions 3, 4 et 5 du groupe parent phényle peuvent être substitués dans le groupe hétérocyclique correspondant par Br, $CF_3$, $CH_3$, Cl, CN, F, I, $NH_2$, $NO_2$, $OCH_3$ ou OH,

. les groupes carbocycliques indan-5-yle, indèn-2-yle, indèn-5-yle, indèn-6-yle et les groupes hétérocycliques correspondants obtenus par remplacement de 1 à 4 atomes de carbone du cycle parent hydrocarboné par 1 à 4 atomes d'azote, d'oxygène ou de soufre, ou par les groupes SO, CO et $SO_2$ pour les carbones 1 et 3 du groupe parent indan-5-yle ou par les groupes NO ou $CCH_3$ pour le carbone 3 du groupe parent 1*H*-indèn-5-yle ou pour les carbones 1 et 3 du groupe parent 2*H*-indèn-5-yle,

et dans lesquels l'atome d'hydrogène de la position 7 du groupe indan (ou indèn)-5-yle et de la position 4 du groupe indèn-6-yle peut être substitué dans le groupe carbocyclique et dans le groupe hétérocyclique correspondant par Br, $CF_3$, $CH_3$, Cl, CN, F, I, $NH_2$, $NO_2$, $OCH_3$ ou OH,

. les groupes carbocycliques 2-naphtyle, 5,6,7,8-tétrahydro-2-naphtyle et les groupes hétérocycliques correspondants obtenus par remplacement de 1 à 4 atomes de carbone du cycle parent hydrocarboné par 1 à 4 atomes d'azote, d'oxygène ou de soufre, ou par les groupes SO, CO et $SO_2$ pour les carbones 5 et 8 du groupe parent 5,6,7,8-tétrahydronaphtyle,

et dans lesquels l'atome d'hydrogène de la position 4 des groupes 2-naphtyle et 5,6,7,8-tétrahydro-2-naphtyle peut être substitué, aussi bien dans le groupe carbocyclique que dans le groupe hétérocyclique correspondant, par Br, $CF_3$, $CH_3$, Cl, CN, F, I, $NH_2$, $NO_2$, $OCH_3$ ou OH ;

– à condition, quand A = N, que $R_2$ ne soit pas un groupe

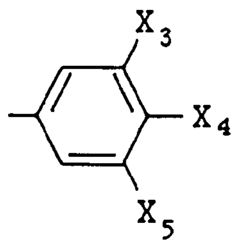

dans lequel $X_3$, $X_4$ et $X_5$ sont :

H,
Br,
$CF_3$,
$CF_2CF_3$,
$CH_2CF_3$,
$C_1$-$C_4$ alkyl,
$CH{=}NOCH_3$,
CH=NOH,
CHO,
$CH_2OCH_3$,
$CH_2OH$,
Cl,
CN,
$COCF_3$,
$COC_1$-$C_3$ alkyl,
$CONH_2$,
$CONHC_1$-$C_3$ alkyl,
$CON(C_1$-$C_3$ alkyl$)_2$,
$COOC_1$-$C_3$ alkyl,
COOH,
F,
I,
$NH_2$,
$NHC_1$-$C_3$ alkyl,
$N(C_1$-$C_3$ alkyl$)_2$,
NHCHO,
$NHCOCH_3$,
$NHCONH_2$,
$NHSO_2CH_3$,
$NO_2$,
$OC_1$-$C_3$ alkyl,
$OCOCH_3$,
OH,
$SC_1$-$C_3$ alkyl,
$SOC_1$-$C_3$ alkyl,
$SO_2C_1$-$C_3$ alkyl,
$SO_2NH_2$,
$SO_2NHC_1$-$C_3$ alkyl,
$SO_2N(C_1$-$C_3$ alkyl$)_2$ et
$SO_3H$.

2. Agents édulcorants selon la revendication 1 caractérisés en ce que les hétéroatomes qui, dans les cycles parents hydrocarbonés remplacent les atomes de carbone pour former un groupe hétérocyclique $R_o$ correspondant sont :

. N pour remplacer CH, et
. NH, O, S, SO et $SO_2$ pour remplacer $CH_2$.

3. Agents édulcorants selon la revendication 2 caractérisés en ce que $R_o$, quand $R_o$ est un groupe hétérocyclique, est choisi dans le groupe comprenant un cycle pyrrolyle, pyrazolyle, imidazolyle, furyle, oxazolyle, isoxazolyle, oxadiazolyle, furazanyle, thiényle, thiazolyle, isothiazolyle, pyridyle, 2-cyanopyridyle, pyridazinyle,

pyrimidinyle, 2-cyanopyrimidinyle, pyrazinyle, indolyle, isoindolyle, indolizinyle, indazolyle, purinyle, isobenzofuranyle, 3,4-méthylènedioxyphényle, benzisoxazolyle, benzofurazanyle, benzothiazolyle, saccharin-5-yle, saccharin-6-yle, quinolyle, isoquinolyle, cinnolinyle, phtalazinyle, quinazolinyle, quinoxalinyle, naphtyridinyle, 1,5-naphtyridinyle, 1,6-naphtyridinyle, 1,7-naphtyridinyle, 1,3,5-triazanaphtyle, 1,4,5-triazanaphtyle, 1,2,8-triazanaphtyle, 1,3,8-triazanaphtyle, 1,2,5-triazanaphtyle, ptéridinyle ou isocoumarinyle.

4. Agents édulcorants selon la revendication 1 caractérisés en ce que A est un atome d'azote.

5. Agents édulcorants selon l'une des revendications 1 à 4, caractérisés en ce que $R_1$ est choisi dans le groupe constitué par H et $CH_3$.

6. Agents édulcorants selon l'une des revendications 1 à 5, caractérisés en ce que $R_2$ est choisi dans le groupe constitué par:

$C_2$-$C_{13}$ alk(én)(yn)yl normal,
$C_3$-$C_{13}$ alk(én)(yn)yl ramifié,
$C_3$-$C_{13}$ cycloalkalk(én)yl,
$C_4$-$C_{13}$ alk(én)yl cycloalk(én)yl,
$C_4$-$C_{13}$ cycloalk(én)yl alk(én)yl,
$C_5$-$C_{13}$ alk(én)yl cycloalk(én)yl alk(én)yl,
$C_7$-$C_{13}$ alk(én)yl bicycloalk(én)yl,
$C_7$-$C_{13}$ bicycloalk(én)yl fusionné,
$C_8$-$C_{13}$ alk(én)yl bicycloalk(én)yl fusionné,
$C_8$-$C_{13}$ bicycloalk(én)yl fusionné alk(én)yl,
$C_9$-$C_{13}$ alk(én)yl bicycloalk(én)yl fusionné alk(én)yl,
$C_{10}$-$C_{13}$ tricycloalk(én)yl fusionné,
$C_{11}$-$C_{13}$ alk(én)yl tricycloalk(én)yl fusionné,
$C_{11}$-$C_{13}$ tricycloalk(én)yl fusionné alk(én)yl,
et $C_{13}$ alk(én)yl tricycloalk(én)yl fusionné alk(én)yl.

7. Agents édulcorants selon la revendication 6 caractérisés en ce que $R_2$ est un groupe hydrocarboné modifié dans lequel :

. jusqu'à 4 atomes de carbone peuvent être remplacés par des hétéroatomes, identiques ou différents, choisis dans le groupe constitué par :

S pour remplacer C ou $CH_2$,
N pour remplacer CH,
NH et O pour remplacer $CH_2$, et
Cl, Br et I pour remplacer $CH_3$,

. et jusqu'à 5 atomes d'hydrogène peuvent être substitués par des atomes de fluor.

8. Agents édulcorants selon l'une des revendications 1 à 7, caractérisés en ce que $R_3$, dans l'une des formes tautomères de la molécule, est un atome d'hydrogène.

9. Agents édulcorants selon l'une des revendications 1 à 8, caractérisés en ce que $R_4$ est un atome d'hydrogène.

10. Agents édulcorants selon la revendication 1 caractérisés en ce qu'ils consistent en des composés de formule :

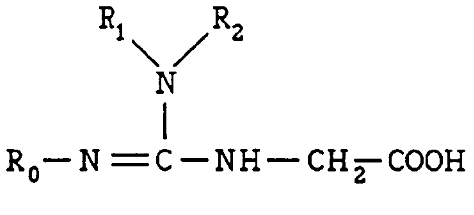

11. Agents édulcorants selon la revendication 10 caractérisés en ce que $R_1$ est H ou $CH_3$.

12. Agents édulcorants selon les revendications 10 et 11, caractérisés en ce que $R_2$ est c-$C_8H_{15}$, $CH_2C_6H_5$, $CH_2$-*c*-$C_6H_{11}$, $CH(CH_3)C_6H_5$ ou $CH(CH_3)$-*c*-$C_6H_{11}$.

13. Agents édulcorants selon les revendications 10, 11 et 12, caractérisés en ce que $R_o$ est choisi dans le groupe constitué par un groupe 2-cyanopyrid-5-yle, 2-cyanopyrimidin-5-yle, indazol-6-yle, benzisoxazol-5-yle, benzisoxazol-6-yle, benzofurazan-5-yle, isoquinol-6-yle, quinazolin-7-yle, 1,7-naphtyridin-3-yle ou 1,3,5-triazanapht-7-yle.

14. Agents édulcorants selon les revendications 1 à 13, caractérisés en ce que ces composés sont salifiés

par des acides ou des bases inorganiques ou organiques physiologiquement acceptables.

15. Agents édulcorants selon la revendication 14 caractérisés en ce que ces composés sont salifiés sous forme d'hydrochlorure ou de sels de sodium, potassium, ammonium, calcium et magnésium.

16. Procédé pour édulcorer les aliments, boissons, confiseries, pâtisseries, chewing gum, produits d'hygiène, cosmétiques, articles de toilette, produits pharmaceutiques et vétérinaires, caractérisé en ce qu'il consiste à ajouter à ces produits une quantité adéquate d'au moins un agent édulcorant selon l'une des revendications 1 à 15.

17. Préparations édulcorées suivant le procédé de la revendication 16.

18. Compositions édulcorantes caractérisées en ce qu'elles comprennent une quantité adéquate d'un ou plusieurs agents édulcorants selon l'une des revendications 1 à 15 et un support ou agent de charge choisi dans le groupe comprenant le polydextrose, l'amidon, les maltodextrines, la cellulose, la méthylcellulose, la carboxyméthylcellulose, l'hydroxyméthylcellulose, la cellulose microcristalline, l'alginate de sodium, les pectines, les gommes, le lactose, le maltose, le glucose, la leucine, le glycérol, le mannitol, le sorbitol, le bicarbonate de sodium et les acides phosphorique, citrique, tartrique, fumarique, benzoïque, sorbique et propionique, et leurs sels de sodium, potassium et calcium.

19. Compositions édulcorantes caractérisées en ce qu'elles comprennent un agent édulcorant selon l'une des revendications 1 à 15 et au moins un autre agent édulcorant, l'autre agent édulcorant étant choisi dans le groupe comprenant le saccharose, le sirop de maïs, le fructose, l'aspartame, l'alitame, la néohespéridine dihydrochalcone, l'isomaltulose hydrogéné, le stévioside, les sucres L, la glycyrrhizine, le xylitol, le sorbitol, le mannitol, l'acésulfame-K, la saccharine et ses sels de sodium, potassium, ammonium ou calcium, l'acide cyclamique et ses sels de sodium, potassium, ou calcium, le trichlorogalactosucrose, la monelline, la thaumatine.

**Revendications pour les Etats contractants suivants : ES, GR**

1. Procédé pour préparer un agent édulcorant de formule générale :

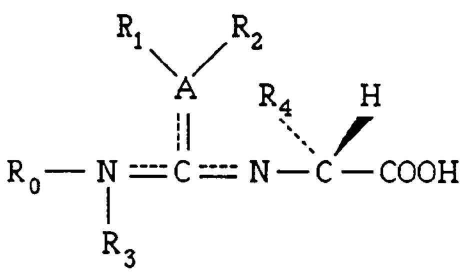

en incluant les formes tautomères et les sels physiologiquement acceptables,

– dans laquelle $R_0$ est choisi dans le groupe comprenant :

. les groupes hétérocycliques dérivés des groupes parents hydrocarbonés 1,3-cyclopentadièn-1-yle, 2,4-cyclopentadièn-1-yle et phényle par remplacement de 1 à 3 atomes de carbone du cycle parent hydrocarboné par 1 à 3 atomes d'azote, d'oxygène et de soufre,
et dans lesquels les atomes d'hydrogène des positions 3 et 4 des groupes parents 1,3-cyclopentadièn-1-yle, 2,4-cyclopentadièn-1-yle et des positions 3, 4 et 5 du groupe parent phényle peuvent être substitués dans le groupe hétérocyclique correspondant par Br, $CF_3$, $CH_3$, Cl, CN, F, I, $NH_2$, $NO_2$, $OCH_3$ ou OH,

. les groupes carbocycliques indan-5-yle, indèn-2-yle, indèn-5-yle, indèn-6-yle et les groupes hétérocycliques correspondants obtenus par remplacement de 1 à 4 atomes de carbone du cycle parent hydrocarboné par 1 à 4 atomes d'azote, d'oxygène ou de soufre, ou par les groupes SO, CO et $SO_2$ pour les carbones 1 et 3 du groupe parent indan-5-yle ou par les groupes NO ou $CCH_3$ pour le carbone 3 du groupe parent 1*H*-indèn-5-yle ou pour les carbones 1 et 3 du groupe parent 2*H*-indèn-5-yle,
et dans lesquels l'atome d'hydrogène de la position 7 du groupe indan(ou indèn)-5-yle et de la position 4 du groupe indèn-6-yle peut être substitué dans le groupe carbocyclique et dans le groupe hétérocyclique correspondant par Br, $CF_3$, $CH_3$, Cl, CN, F, I, $NH_2$, $NO_2$, $OCH_3$ ou OH,

. les groupes carbocycliques 2-naphtyle, 5,6,7,8-tétrahydro-2-naphtyle et les groupes hétéro-cycliques correspondants obtenus par remplacement de 1 à 4 atomes de carbone du cycle parent hydrocarboné par 1 à 4 atomes d'azote, d'oxygène, de soufre et par les groupes SO, CO et $SO_2$ pour les carbones 5 et 8 du groupe parent 5,6,7,8-tétrahydronaphtyle,

et dans lesquels l'atome d'hydrogène de la position 4 des groupes 2-naphtyle et 5,6,7,8-tétrahydro-2-naphtyle peut être substitué, aussi bien dans le groupe carbocyclique que dans le groupe hétérocyclique correspondant, par Br, $CF_3$, $CH_3$, Cl, CN, F, I, $NH_2$, $NO_2$, $OCH_3$ ou OH.

à condition, quand A = N, que $R_2$ ne soit pas un groupe

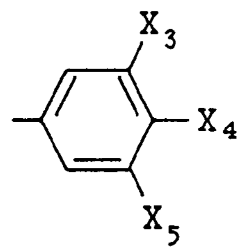

dans lequel $X_3$, $X_4$ et $X_5$ sont :

H,
Br,
$CF_3$,
$CF_2CF_3$,
$CH_2CF_3$,
$C_1$-$C_4$ alkyl,
$CH=NOCH_3$,
CH=NOH,
CHO,
$CH_2OCH_3$,
$CH_2OH$,
Cl,
CN,
$COCF_3$,
$COC_1$-$C_3$ alkyl,
$CONH_2$,
$CONHC_1$-$C_3$ alkyl,
$CON(C_1$-$C_3$ alkyl$)_2$,
$COOC_1$-$C_3$ alkyl,
COOH,
F,
I,
$NH_2$,
$NCH_1$-$C_3$ alkyl,
$N(C_1$-$C_3$ alkyl$)_2$,
NHCHO,
$NHCOCH_3$,
$NHCONH_2$,
$NHSO_2CH_3$,
$NO_2$,
$OC_1$-$C_3$ alkyl,
$OCOCH_3$,
OH,
$SC_1$-$C_3$ alkyl,
$SOC_1$-$C_3$ alkyl,
$SO_2C_1$-$C_3$ alkyl,
$SO_2NH_2$,
$SO_2NHC_1$-$C_3$ alkyl,
$SO_2N(C_1$-$C_3$ alkyl$)_2$ et
$SO_3H$ ;

– dans laquelle A est choisi dans le groupe comprenant N et C ;

– dans laquelle $R_1$ est un atome d'hydrogène ou un groupe hydrocarboné ou un groupe hydrocarboné

modifié, ayant jusqu'à 4 atomes de carbone, saturé ou insaturé, acyclique, cyclique ou mixte, et dans lequel, dans le groupe hydrocarboné modifié :

. 1 à 2 atomes de carbone peuvent être remplacés par 1 à 2 hétéroatomes, identiques ou différents, choisis dans le groupe comprenant les atomes d'azote, oxygène, soufre, chlore, brome et iode,

. et 1 à 3 atomes d'hydrogène peuvent être remplacés par 1 à 3 atomes de fluor ;

– dans laquelle $R_2$ est un groupe hydrocarboné ou un groupe hydrocarboné modifié, de 2 à 13 atomes de carbone, saturé ou insaturé, acyclique, cyclique ou mixte, et dans lequel, dans le groupe hydrocarboné modifié :

. 1 à 4 atomes de carbone peuvent être remplacés par 1 à 4 hétéroatomes, identiques ou différents, choisis dans le groupe comprenant les atomes d'azote, oxygène, soufre, chlore, brome et iode,

. et 1 à 5 atomes d'hydrogène peuvent être remplacés par 1 à 5 atomes de fluor ;

– dans laquelle $R_1$ et $R_2$ peuvent être fusionnés ;

– dans laquelle $R_3$ est choisi dans le groupe comprenant un atome d'hydrogène ou un groupe alkyle $C_1$-$C_3$ ;

– dans laquelle $R_4$ est choisi dans le groupe comprenant :

H et

$CH_3$;

caractérisé en ce qu'il consiste à condenser un dérivé thiouréido ou thioamido de formule générale :

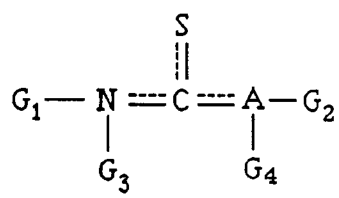

avec un composé de formule générale :

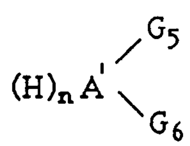

dans lesquelles :

. $G_1$ est $R_0$ ou $HOOCCHR_4$,

. $G_2$ est $R_2$ ou $HOOCCHR_4$,

. $G_3$, $G_4$ et $G_5$ sont H, $R_1$ ou $R_3$,

. $G_6$ est $R_0$, $R_2$ ou $HOOCCHR_4$,

. A' est N ou C,

. n est égal à 1 quand A' est N, et est égal à 2 quand A' est C ;

et dans lesquelles :

. $G_1$, $G_2$ et $G_6$ ne sont pas simultanément identiques,

. A et A' ne sont pas simultanément un atome de carbone,

. A' est N et $G_3$, $G_4$ et $G_5$ sont H lorsque $G_1$ ou $G_2$ ou $G_6$ sont $HOOCCHR_4$ ;

et dans lesquelles $R_0$, $R_1$, $R_2$, $R_3$, $R_4$ et A correspondent aux définitions données précédemment.

2. Procédé selon la revendication 1 caractérisé en ce que les hétéroatomes qui, dans les cycles parents hydrocarbonés remplacent les atomes de carbone pour former un groupe hétérocyclique $R_0$ correspondant sont :

. N pour remplacer CH, et

. NH, O, S, SO et $SO_2$ pour remplacer $CH_2$.

3. Procédé selon la revendication 1 caractérisé en ce que $R_0$, quand $R_0$ est un groupe hétérocyclique, est choisi dans le groupe comprenant un cycle pyrrolyle, pyrazolyle, imidazolyle, furyle, oxazolyle, isoxazolyle, oxadiazolyle, furazanyle, thiényle, thiazolyle, isothiazolyle, pyridyle, 2-cyanopyridyle, pyridazinyle, pyrimidinyle, 2-cyanopyrimidinyle, pyrazinyle, indolyle, isoindolyle, indolizinyle, indazolyle, purinyle, isobenzofuranyle, 3,4-méthylènedioxyphényle, benzisoxazolyle, benzofurazanyle, benzothiazolyle, saccharin-5-yle, saccharin-6-yle, quinolyle, isoquinolyle, cinnolinyle, phtalazinyle, quinazolinyle, quinoxalinyle, naphtyridinyle, 1,5-naphtyridinyle, 1,6-naphtyridinyle, 1,7-naphtyridinyle, 1,3,5-triazanaphtyle, 1,4,5-triazanaphtyle,

1,2,8-triazanaphtyle, 1,3,8-triazanaphtyle, 1,2,5-triazanaphtyle, ptéridinyle ou isocoumarinyle.

4. Procédé selon la revendication 1 caractérisés en ce que :

– A est un atome d'azote;

– $R_1$ est choisi dans le groupe constitué par H et $CH_3$;

– $R_2$ est choisi dans le groupe constitué par:

$C_2$-$C_{13}$ alk(én)(yn)yl normal,
$C_3$-$C_{13}$ alk(én)(yn)yl ramifié,
$C_3$-$C_{13}$ cycloalk(én)yl,
$C_4$-$C_{13}$ alk(én)yl cycloalk(én)yl,
$C_4$-$C_{13}$ cycloalk(én)yl alk(én)yl,
$C_5$-$C_{13}$ alk(én)yl cycloalk(én)yl alk(en)yl,
$C_7$-$C_{13}$ alk(én)yl bicycloalk(én)yl,
$C_7$-$C_{13}$ bicycloalk(én)yl fusionné,
$C_8$-$C_{13}$ alk(én)yl bicycloalk(én)yl fusionné,
$C_8$-$C_{13}$ bicycloalk(én)yl fusionné alk(én)yl,
$C_9$-$C_{13}$ alk(én)yl bicycloalk(én)yl fusionné alk(én)yl,
$C_{10}$-$C_{13}$ tricycloalk(én)yl fusionné,
$C_{11}$-$C_{13}$ alk(én)yl tricycloalk(én)yl fusionné,
$C_{11}$-$C_{13}$ tricycloalk(én)yl fusionné alk(én)yl,
et $C_{13}$ alk(én)yl tricycloalk(én)yl fusionné alk(én)yl;

– $R_3$, dans l'une des formes tautomères de la molécule, est un atome d'hydrogène;

– $R_4$ est un atome d'hydrogène.

5. Procédé selon la revendication 1 caractérisé en ce que l'on prépare un composé de formule :

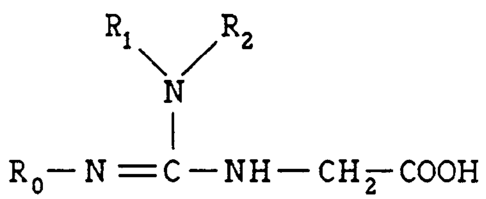

dans laquelle :

– $R_o$ est choisi dans le groupe constitué par un groupe 2-cyanopyrid-5-yle, 2-cyanopyrimidin-5-yle, indazol-6-yle, benzisoxazol-5-yle, benzisoxazol-6-yle, benzofurazan-5-yle, isoquinol-6-yle, quinazolin-7-yle, 1,7-naphtyridin-3-yle ou 1,3,5-triazanapht-7-yle,

– $R_1$ est H ou $CH_3$,

– $R_2$ est c-$C_8H_{15}$, $CH_2C_6H_5$, $CH_2$-*c*-$C_6H_{11}$, $CH(CH_3)C_6H_5$ ou $CH(CH_3)$-*c*-$C_6H_{11}$.

6. Procédé selon la revendication 1, caractérisé en ce qu'il consiste à obtenir le dérivé thiouréido par l'action d'un isothiocyanate sur une amine selon l'une des réactions :

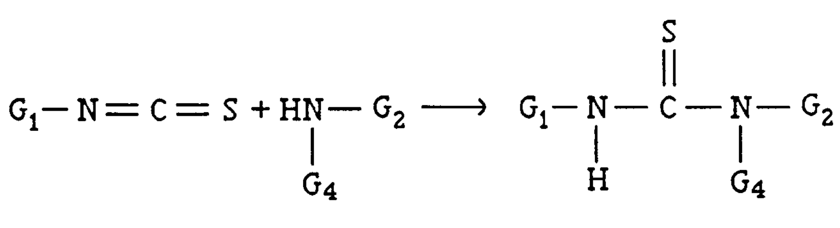

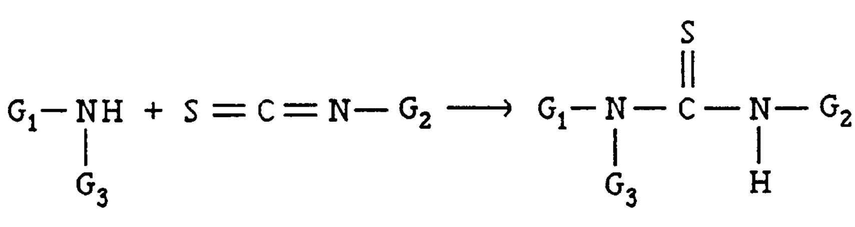

7. Procédé selon la revendication 1, caractérisé en ce qu'il consiste à activer l'atome de soufre du dérivé thiouréido ou thioamido en le transformant en un groupe S-alkyle ou $SO_3H$, ou en le remplaçant par un groupe O-alkyl, O-$SO_2$aryl ou un atome d'halogène.

8. Procédé selon les revendications 1 et 7, caractérisé en ce qu'il consiste à activer le dérivé thiouréido ou thioamido en le transformant en un dérivé S-méthylisothiouréido ou S-méthylisothioamido de formule générale :

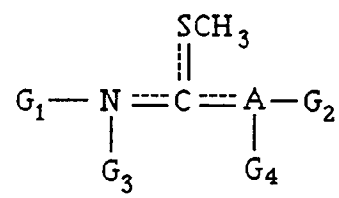

par action d'un agent alkylant choisi dans le groupe comprenant l'iodure de méthyle et le diméthyl sulfate.

9. Procédé selon la revendication 8, caractérisé en ce que le dérivé S-méthylisothiouréido correspond à la formule générale :

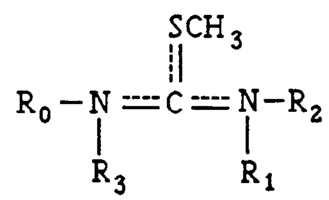

10. Procédé selon la revendication 1, caractérisé en ce qu'il consiste à activer le dérivé thiouréido, lorsque A est N et lorsque $G_3$ et $G_4$ sont un atome d'hydrogène, en le transformant en un intermédiaire carbodiimide :

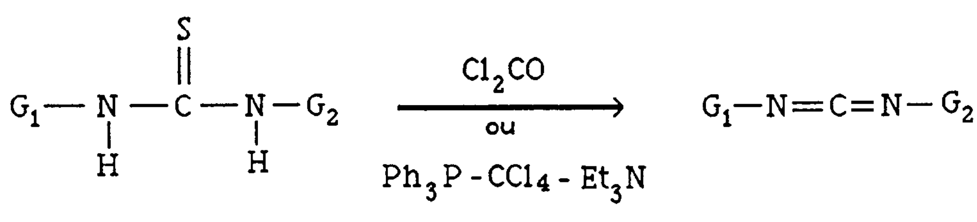


par traitement par le phosgène ou par action d'un mélange équimoléculaire de $(C_6H_5)_3P$ - $CCl_4$ - $(C_2H_5)_3N$.

11. Procédé selon la revendication 1, caractérisé en ce que, lorsque A et A' sont N et qu'au moins l'un des radicaux $G_3$, $G_4$ ou $G_5$ est un atome d'hydrogène, le dérivé thiouréido est condensé avec l'amine par action de dicyclohexylcarbodiimide (DCC) comme agent de couplage, suivant la réaction :

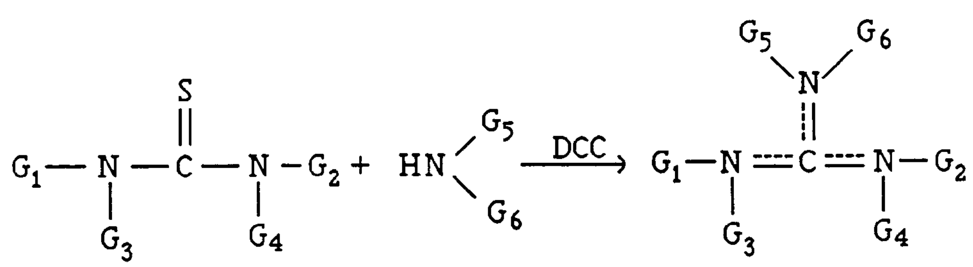

## Patentansprüche

### Patentansprüche für folgende Vertragsstraaten : AT, BE, CH, LI, DE, FR, GB, IT, LU, NL, SE

1. Süßstoffe mit der allgemeinen Formel

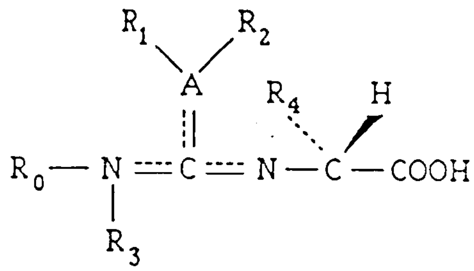

wobei die tautomeren Formen und die physiologisch akzeptablen Salze eingeschlossen sind,

– worin A unter N und C ausgewählt wird,

– worin $R_1$ ein Wasserstoffatom oder eine Kohlenwasserstoffgruppe oder eine modifizierte Kohlenwasserstoffgruppe mit bis zu 4 Kohlenstoffatomen ist, die gesättigt oder ungesättigt, acyclisch, cyclisch oder gemischt sein kann und wobei in der modifizierten Kohlenwasserstoffgruppe,

. 1 bis 2 Kohlenstoffatome durch 1 bis 2 gleiche oder verschiedene Heteroatome, die unter Stickstoff, Sauerstoff, Schwefel, Chlor, Brom und Jod ausgewählt werden, ersetzt sein können,

. 1 bis 3 Wasserstoffatomen durch 1 bis 3 Fluoratome ersetzt sein können,

– worin $R_2$ eine Kohlenwasserstoffgruppe oder eine modifizierte Kohlenwasserstoffgruppe mit 2 bis 13 Kohlenstoffatomen ist, die gesättigt oder ungesättigt, acyclisch, cyclisch oder gemischt sein kann und wobei in der modifizierten Kohlenwasserstoffgruppe

. 1 bis 4 Kohlenstoffatome durch 1 bis 4 gleiche oder verschiedene Heteroatome ersetzt sein können, die ausgewählt werden unter Stickstoff, Sauerstoff, Schwefel, Chlor, Brom und Jod,

. 1 bis 5 Wasserstoffatome durch 1 bis 5 Fluoratome ersetzt sein können,

– worin $R_1$ und $R_2$ verknüpft sein können,

– $R_3$ unter einem Wasserstoffatom oder einem $C_1$-$C_3$ Alkylrest und,

– $R_4$ unter

H und

$CH_3$

ausgewählt wird,

dadurch gekennzeichnet, daß $R_0$ ausgewählt unter:

. den heterocyclischen Gruppen, die von den Kohlenwasserstoffgruppen 1,3-Cyclopentadien-1-yl, 2,4-Cyclopentadien-1-yl und Phenyl durch Ersetzen von 1 bis 3 Kohlenstoffatomen des cyclischen Kohlenwasserstoffs durch 1 bis 3 Stickstoffatome, Sauerstoffatome und Schwefelatome abgeleitet sind, und worin die Wasserstoffatome der Positionen 3 und 4 der 1,3-Cyclopentadien-1-yl-Gruppe, 2,4-Cyclopentadien-1-yl-Gruppe und der Positionen 3, 4 und 5 der Phenylgruppe in der entsprechenden heterocyclischen Gruppe durch Br, $CF_3$, $CH_3$, Cl, CN, F, I, $NH_2$, $NO_2$, $OCH_3$ oder OH substituiert sein können,

. den carbocyclischen Gruppen Indan-5-yl, Inden-2-yl, Inden-5-yl, Inden-6-yl und den entsprechenden heterocyclischen Gruppen, die durch Ersetzen von 1 bis 4 Kohlenstoffatomen des entsprechenden cyclischen Kohlenwasserstoffs durch 1 bis 4 Stickstoffatome, Sauerstoffatome oder Schwefelatome oder durch SO, CO und $SO_2$ für die Kohlenstoffatome 1 und 3 der Indan-5-yl-Gruppe oder durch NO oder $CCH_3$ für das Kohlenstoffatom 3 der 1H-Inden-S-yl-Gruppe oder für die Kohlenstoffatome 1 und 3 der 2H-Inden-5-yl-Gruppe erhalten werden,
und worin das Wasserstoffatom der Position 7 der Indan(oder Inden)-5-yl-Gruppe und der Position 4 der Inden-6-yl-Gruppe in der carbocyclischen Gruppe und der entsprechenden heterocyclischen Gruppe durch Br, $CF_3$, $CH_3$, Cl, CN, F, I, $NH_2$, $NO_2$ $OCH_3$ oder OH substituiert sein können,

. den carbocyclischen Gruppen 2-Naphtyl, 5,6,7,8-Tetrahydro-2-naphtyl und den entsprechenden heterocyclischen Gruppen, die durch Ersetzen von 1 bis 4 Kohlenstoffatomen des cyclischen Kohlenwasserstoffs durch 1 bis 4 Stickstoffatome, Sauerstoffatome oder Schwefelatome oder durch SO, CO und $SO_2$ für die Kohlenstoffatome 5 und 8 der 5,6,7,8-Tetrahydronaphthylgruppe erhalten werden,
und worin das Wasserstoffatom der Position 4 der 2-Naphthylgruppe und der 5,6,7,8-Tetrahydro-2-naphthylgruppe ebenso in der carbocyclischen Gruppe wie auch in der entsprechenden heterocy-

clischen Gruppe durch Br, $CF_3$, $CH_3$, Cl, CN, F, I, $NH_2$, $NO_2$, $OCH_3$ oder OH substituiert sein können,
– unter der Bedingung, daß, wenn A = N, $R_2$ keine Gruppe mit der Formel

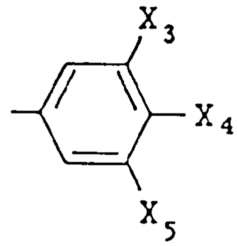

ist, worin $X_3$, $X_4$ und $X_5$ sind:

H,
Br,
$CF_3$,
$CF_2CF_3$,
$CH_2CF_3$,
$C_1$-$C_4$ -Alkyl,
$CH{=}NOCH_3$,
CH=NOH,
CHO,
$CH_2OCH_3$,
$CH_2OH$,
Cl,
CN,
$COCF_3$,
$COC_1$-$C_3$ -Alkyl,
$CONH_2$,
$CONHC_1$-$C_3$-Alkyl,
CON ($C_1$-$C_3$-Alkyl) $_2$,
$COOC_1$-$C_3$-Alkyl,
COOH,
F,
I,
$NH_2$,
$NHC_1$-$C_3$-Alkyl,
N($C_1$-$C_3$Alkyl)$_2$,
NHCHO,
$NHCOCH_3$,
$NHCONH_2$,
$NHSO_2CH_3$,
$NO_2$ ,
$OC_1$-$C_3$Alkyl,
$OCOCH_3$,
OH,
$SC_1$-$C_3$Alkyl,
$SOC_1$-$C_3$ Alkyl,
$SO_2C_1$-$C_3$Alkyl,
$SO_2NH_2$,
$SO_2NHC_1$-$C_3$-Alkyl,
$SO_2N$ ($C_1$-$C_3$-Alkyl) $_2$ und
$SO_3H$.

Süßstoffe nach Anspruch 1, dadurch gekennzeichnet, daß die Heteroatome, die in den cyclischen Kohlenwasserstoffen die Kohlenstoffatome unter Bildung einer heterocyclischen Gruppe $R_0$ ersetzen, sind:

. N zum Ersatz von CH und
. NH, O, S, SO und $SO_2$ zum Ersatz von $CH_2$.

3. Süßstoffe nach Anspruch 2, dadurch gekennzeichnet, daß $R_0$, wenn $R_0$ eine heterocyclische Gruppe ist, ausgewählt wird unter Pyrrolyl, Pyrazolyl, Imidazolyl, Furyl, Oxazolyl, Isoxazolyl, Oxadiazolyl, Furazanyl, Thienyl, Thiazolyl, Isothiazolyl, Pyridyl, 2-Cyanopyridyl, Pyridazinyl, Pyrimidinyl, 2-Cyanopyrimidinyl, Pyrazinyl, Indolyl, Isoindolyl, Indolizinyl, Indazolyl, Purinyl, Isobenzofuranyl, 3,4-Methylendioxyphenyl, Benzisoxazolyl, Benzofurazanyl, Benzothiazolyl, Saccharin-5-yl, Saccharin-6-yl, Chinolyl, Isochinolyl, Cinnolinyl, Phthalazinyl, Chinazolinyl, Chinoxalinyl, Naphthyridinyl, 1,5-Naphthyridinyl, 1,6-Naphthyridinyl, 1,7-Naphthyridinyl, 1,3,5-Triazanaphthyl, 1,4,5-Triazanaphthyl, 1,2,8-Triazanaphthyl, 1,3,8-Triazanaphthyl, 1,2,5-Triazanaphthyl, Pteridinyl oder Isocumarinyl.

4. Süßstoffe nach Anspruch 1, dadurch gekennzeichnet, daß A ein Stickstoffatom ist.

5. Süßstoffe nach einem der Ansprüche 1-4, dadurch gekennzeichnet, daß $R_1$ unter H und $CH_3$ ausgewählt wird.

6. Süßstoffe nach einem der Ansprüche 1-5, dadurch gekennzeichnet, daß $R_2$ ausgewählt wird unter

$C_2$-$C_{13}$-Alk(en)(in)yl geradkettig,
$C_3$-$C_{13}$-Alk(en)(in)yl verzweigt,
$C_3$-$C_{13}$-Cycloalk(en)yl,
$C_4$-$C_{13}$-Alk(en)yl Cycloalk(en)yl,
$C_4$-$C_{13}$-Cycloalk(en)yl Alk(en)yl,
$C_5$-$C_{13}$-Alk(en)yl Cycloalk(en)yl Alk(en)yl,
$C_7$-$C_{13}$-Alk(en)yl Bicycloalk(en)yl,
$C_7$-$C_{13}$-Bicycloalk(en)yl verknüpft,
$C_8$-$C_{13}$-Alk(en)yl Bicycloalk(en)yl verknüpft,
$C_8$-$C_{13}$-Bicycloalk(en)yl verknüpft Alk(en)yl,
$C_9$-$C_{13}$-Alk(en)yl Bicycloalk(en)yl verknüpft Alk(en)yl,
$C_{10}$-$C_{13}$-Tricycloalk(en)yl verknüpft,
$C_{11}$-$C_{13}$-Alk(en)yl Tricycloalk(en)yl verknüpft,
$C_{11}$-$C_{13}$-Tricycloalk(en)yl verknüpft Alk(en)yl
und $C_{13}$ Alk(en)yl Tricycloalk(en)yl verknüpft Alk(en)yl.

7. Süßstoffe nach Anspruch 6, dadurch gekennzeichnet, daß $R_2$ eine modifizierte Kohlenwasserstoffgruppe ist, in der

. bis zu 4 Kohlenstoffatome durch gleiche oder verschiedene Heteroatome ersetzt sein können, die ausgewählt werden unter

S zum Ersatz von C oder $CH_2$
N zum Ersatz von CH,
NH und O zum Ersatz von $CH_2$, und
Cl, Br und I zum Ersatz von $CH_3$,

. und bis zu 5 Wasserstoffatome durch Fluoratome substituiert sein können.

8. Süßstoffe nach einem der Ansprüche 1-7, dadurch gekennzeichnet, daß $R_3$ in einer der tautomeren Formen des Moleküls ein Wasserstoffatom ist.

9. Süßstoffe nach einem der Ansprüche 1-8, dadurch gekennzeichnet, daß $R_4$ ein Wasserstoffatom ist.

10. Süßstoffe nach Anspruch 1, dadurch gekennzeichnet, daß sie aus Verbindungen der Formel

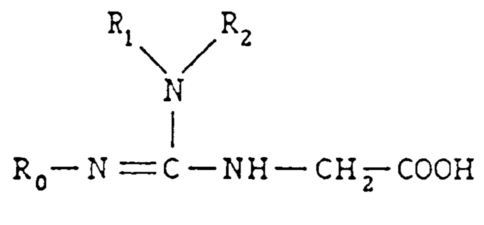

bestehen.

11. Süßstoffe nach Anspruch 10, dadurch gekennzeichnet, daß $R_1$ H oder $CH_3$ ist.

12. Süßstoffe nach den Ansprüchen 10 und 11, dadurch gekennzeichnet, daß $R_2$ c-$C_8H_{15}$, $CH_2C_6H_5$, $CH_2$-c-$C_6H_{11}$, $CH(CH_3)C_6H_5$ oder $CH(CH_3)$-c-$C_6H_{11}$ ist.

13. Süßstoffe nach den Ansprüchen 10, 11 und 12, dadurch gekennzeichnet, daß $R_0$ ausgewählt wird unter 2-Cyanopyrid-5-yl, 2-Cyanopyrimidin-5-yl, Indazol-6-yl, Benzisoxazol-5-yl, Benzisoxazol-6-yl, Benzofurazan-5-yl, Isochinol-6-yl, Chinazolin-7-yl, 1,7-Naphthyridin-3-yl oder 1,3,5-Triazanaphth-7-yl.

14. Süßstoffe nach den Ansprüchen 1-13, dadurch gekennzeichnet, daß die Verbindungen durch anorganische oder organische physiologisch akzeptable Säuren oder Basen in Salze überführt sind.

15. Süßstoffe nach Anspruch 4, dadurch gekennzeichnet, daß die Verbindungen in das Hydrochlorid oder die Natrium-, Kalium-, Ammonium-, Calcium- und Magnesiumsalze überführt sind.

16. Verfahren zum Süßen mit Süßstoff von Lebensmitteln, Getränken, Zuckerwaren, Backwaren, Kaugummi, Hygieneprodukten, Kosmetika, Toilettenartikeln, pharmazeutischen Produkten und Veterinärprodukten, dadurch gekennzeichnet, daß diesen Produkten eine adäquate Menge von mindestens einem Süßstoff nach einem der Ansprüche 1-15 beigefügt wird.

17. Mit Süßstoff gesüßte Zubereitungen nach dem Verfahren des Anspruchs 16.

18. Zusammensetzungen zum Süßen mit Süßstoff, dadurch gekennzeichnet, daß sie eine adäquate Menge von einem oder mehreren Süßstoffen nach einem der Ansprüche 1-15 und ein Trägermaterial oder einen Füllstoff enthalten, die ausgewählt werden unter Polydextrose, Stärke, Maltodextrinen, Cellulose, Methylcellulose, Carboxymethylcellulose, Hydroxymethylcellulose, mikrokristalliner Cellulose, Natriumalginat, Pectinen, Gummi, Lactose, Maltose, Glucose, Leucin, Glycerol, Mannitol, Sorbitol, Natriumbicarbonat und Phosphorsäure, Zitronensäure, Weinsäure, Fumarsäure, Benzoesäure, Sorbinsäure und Propionsäure, und ihren Salzen mit Natrium, Kalium und Calcium.

19. Zusammensetzungen zum Süßen mit Süßstoff, dadurch gekennzeichnet, daß sie einen Süßstoff nach einem der Ansprüche 1-15 und mindestens eine weitere süßende Substanz enthalten, wobei die andere süßende Substanz ausgewählt wird unter Saccharose, Maissirup, Fructose, Aspartam, Alitam, Neohesperidin, Dihydrochalcon, hydrierter Isomaltulose, Steviosid, den L-Zuckern, Glycyrrhizin, Xylitol, Sorbitol, Mannitol, Acesulfam-K, Saccharin und seinen Natrium-, Kalium-, Amonium- oder Calciumsalzen, Cyclamsäure und ihre Natrium-, Kalium- oder Calciumsalzen, Trichlorgalactosucrose, Monellin und Thaumatin.

**Patentansprüche für folgenden Vertragsstaaten : ES, GR**

1. Verfahren zur Herstellung eines Süßstoffes mit der allgemeinen Formel

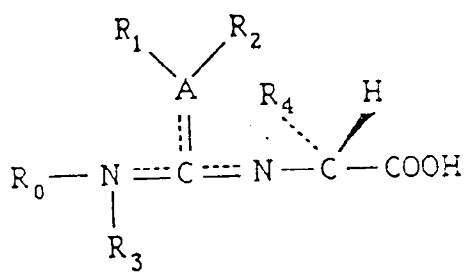

wobei die tautomeren Formen und die physiologisch akzeptablen Salze eingeschlossen sind,

– worin $R_0$ ausgewählt wird unter:

. den heterocyclischen Gruppen, die von den Kohlenwasserstoffgruppen 1,3-Cyclopentadien-1-yl, 2,4-Cyclopentadien-1-yl und Phenyl durch Ersetzen von 1 bis 3 Kohlenstoffatomen des cyclischen Kohlenwasserstoffs durch 1 bis 3 Stickstoffatome, Sauerstoffatome und Schwefelatome abgeleitet sind, und worin die Wasserstoffatome der Positionen 3 und 4 der 1,3-Cyclopentadien-1-yl-Gruppe, 2,4-Cyclopentadien-1-yl-Gruppe und der Positionen 3, 4 und 5 der Phenylgruppe in der entsprechenden heterocyclischen Gruppe durch Br, $CF_3$, $CH_3$, Cl, CN, F, I, $NH_2$, $NO_2$, $OCH_3$ oder OH substituiert sein können,

. den carbocyclischen Gruppen Indan-5-yl, Inden-2-yl, Inden-5-yl, Inden-6-yl und den entsprechenden heterocyclischen Gruppen, die durch Ersetzen von 1 bis 4 Kohlenstoffatomen des entsprechenden cyclischen Kohlenwasserstoffs durch 1 bis 4 Stickstoffatome, Sauerstoffatome oder Schwefelatome oder durch SO, CO und $SO_2$ für die Kohlenstoffatome 1 und 3 der Indan-5-yl-Gruppe oder durch NO oder $CCH_3$ für das Kohlenstoffatom 3 der 1H-Inden-5-yl-Gruppe oder für die Kohlenstoffatome 1 und 3 der 2H-Inden-S-yl-Gruppe erhalten werden, und worin das Wasserstoffatom der Position 7 der Indan(oder Inden)-5-yl-Gruppe und der Position 4 der Inden-6-yl-Gruppe in der carbocyclischen Gruppe und der entsprechenden heterocyclischen Gruppe durch Br, $CF_3$, $CH_3$, Cl, CN, F, I, $NH_2$, $NO_2$ $OCH_3$ oder OH substituiert sein können,

. den carbocyclischen Gruppen 2-Naphtyl, 5,6,7,8-Tetrahydro-2-naphtyl und den entsprechenden heterocyclischen Gruppen, die durch Ersetzen von 1 bis 4 Kohlenstoffatomen des cyclischen Kohlenwasserstoffs durch 1 bis 4 Stickstoffatome, Sauerstoffatome oder Schwefelatome oder durch SO, CO und $SO_2$ für die Kohlenstoffatome 5 und 8 der 5,6,7,8-Tetrahydronaphthylgruppe erhalten werden, und worin das Wasserstoffatom der Position 4 der 2-Naphthylgruppe und der 5,6,7,8-Tetrahydro-2-naphthylgruppe ebenso in der carbocyclischen Gruppe wie auch in der entsprechenden heterocy-

clischen Gruppe durch Br, $CF_3$, $CH_3$, Cl, CN, F, I, $NH_2$, $NO_2$, $OCH_3$ oder OH substituiert sein können,
. unter der Bedingung, daß, wenn A = N, $R_2$ keine Gruppe mit der Formel

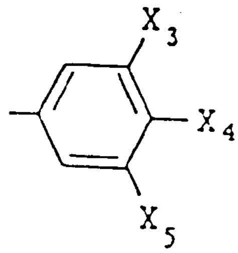

ist, worin $X_3$, $X_4$ und $X_S$ sind:

H,
Br,
$CF_3$,
$CF_2CF_3$,
$CH_2CF_3$,
$C_1$-$C_4$-Alkyl,
$CH{=}NOCH_2$,
CH=NOH,
CHO,
$CH_2OCH_3$,
$CH_2OH$,
Cl,
CN,
$COCF_3$,
$COC_1$-$C_3$ -Alkyl,
$CONH_2$,
$CONHC_1$-$C_3$ -Alkyl,
CON ($C_1$-$C_3$-Alkyl)$_2$,
$COOC_1$-$C_3$-Alkyl,
COOH,
F,
I,
$NH_2$,
$NHC_1$-$C_3$Alkyl,
N($C_1$-$C_3$-Alkyl)$_2$,
NHCHO,
$NHCOCH_3$,
$NHCONH_2$ ,
$NHSO_2CH_3$,
$NO_2$ ,
$OC_1$-$C_3$Alkyl,
$OCOCH_3$,
OH,
$SC_1$-1$C_3$-Alkyl,
$SOC_1$-$C_3$Alkyl,
$SO_2C_1$-$C_3$Alkyl,
$SO_2NH_2$,
$SO_2NHC_1$-$C_3$-Alkyl
$SO_2N$($C_1$-$C_3$-Alkyl)$_2$ und
$SO_3H$

– worin A unter N und C ausgewählt wird,

– worin $R_1$ ein Wasserstoffatom oder ein Kohlenwasserstoffgruppe oder eine modifizierte Kohlenwasserstoffgruppe mit bis zu 4 Kohlenstoffatomen ist, die gesättigt oder ungesättigt, acyclisch, cyclisch oder gemischt sein kann und wobei in der modifizierten Kohlenwasserstoffgruppe,

. 1 bis 2 Kohlenstoffatome durch 1 bis 2 gleiche oder verschiedene Heteroatome, die unter Stickstoff, Sauerstoff, Schwefel, Chlor, Brom und Jod ausgewählt werden, ersetzt sein können,

. 1 bis 3 Wasserstoffatomen durch 1 bis 3 Fluoratome ersetzt sein können,

– worin $R_2$ eine Kohlenwasserstoffgruppe oder eine modifizierte Kohlenwasserstoffgruppe mit 2 bis 13 Kohlenstoffatomen ist, die gesättigt oder ungesättigt, acyclisch, cyclisch oder gemischt sein kann und wobei in der modifizierten Kohlenwasserstoffgruppe

. 1 bis 4 Kohlenstoffatome durch 1 bis 4 gleiche oder verschiedene Heteroatome ersetzt sein können, die ausgewählt werden unter Stickstoff, Sauerstoff, Schwefel, Chlor, Brom und Jod,

. 1 bis 5 Wasserstoffatome durch 1 bis 5 Fluoratome ersetzt sein können,

– worin $R_1$ und $R_2$ verknüpft sein können,

– $R_3$ unter einem Wasserstoffatom oder einer $C_1$-$C_3$ Alkylgruppe und

– $R_4$ unter

H und

$CH_3$

ausgewählt wird,

dadurch gekennzeichnet, daß es darin besteht, ein Thioharnstoffderivat oder Thioamidoderivat mit der allgemeinen Formel

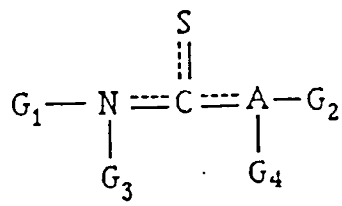

mit einer Verbindung der allgemeinen Formel

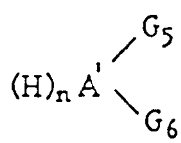

zu kondensieren, worin:

. $G_1$ $R_0$ oder $HOOCCHR_4$,

. $G_2$ $R_2$ oder $HOOCCHR_4$,

. $G_3$, $G_4$ und $G_5$ H, $R_1$ oder $R_3$,

. $G_6$ $R_0$, $R_2$ oder $HOOCCHR_4$,

. A' N oder C ist,

. n = 1, wenn A' = N und

. n = 2, wenn A' = C

und worin

. $G_1$, $G_2$ und $G_6$ nicht gleichteilig identisch sind,

. A und A' nicht gleichteilig ein Kohlenstoffatom sind,

. A' = N und $G_3$, $G_4$ und $G_5$ = H sind, wenn $G_1$, $G_2$ oder $G_6$ $HOOCCHR_4$ sind,

und worin $R_0$, $R_1$, $R_2$, $R_3$, $R_4$und A den vorher gegebenen Definitionen entsprechen.

2. Verfahren nach Anspruch 1, dadurch gekennzeichnet, daß die Heteroatome, die in den cyclischen Kohlenwasserstoffen die Kohlenstoffatome unter Bildung einer heterocyclischen Gruppe $R_0$ ersetzen, sind:

. N zum Ersatz von CH und

. NH, O, S, SO und $SO_2$ zum Ersatz von $CH_2$.

3. Verfahren nach Anspruch 1, dadurch gekennzeichnet, daß $R_0$, wenn $R_0$ eine heterocyclische Gruppe ist, ausgewählt wird unter Pyrrolyl, Pyrazolyl, Imidazolyl, Furyl, Oxazolyl, Isoxazolyl, Oxadiazolyl, Furazanyl, Thienyl, Thiazolyl, Isothiazolyl, Pyridyl, 2-Cyanopyridyl, Pyridazinyl, Pyrimidinyl, 2-Cyanopyrimidinyl, Pyrazinyl, Indolyl, Isoindolyl, Indolizinyl, Indazolyl, Purinyl, Isobenzofuranyl, 3,4-Methylendioxyphenyl, Benzisoxazolyl, Benzofurazanyl, Benzothiazolyl, Saccharin-5-yl, Saccharin-6-yl, Chinolyl, Isochinolyl, Cinnolinyl, Phthalazinyl, Chinazolinyl, Chinoxalinyl, Naphthyridinyl, 1,5-Naphthyridinyl, 1,6-Naphthyridinyl, 1,7-Naphthyridinyl, 1,3,5-Triazanaphthyl, 1,4,5-Triazanaphthyl, 1,2,8-Triazanaphthyl, 1,3,8-Triazanaphthyl, 1,2,5-Triazanaphthyl, Pteridinyl oder Isocumarinyl.

4. Verfahren nach Anspruch 1, dadurch gekennzeichnet, daß

- A ein Stickstoffatom ist,
- $R_1$ unter H und $CH_3$ ausgewählt wird,
- $R_2$ ausgewählt wird unter

  $C_2$-$C_{13}$-Alk(en)(in)yl geradkettig,
  $C_3$-$C_{13}$-Alk(en)(in)yl verzweigt,
  $C_3$-$C_{13}$-Cycloalk(en)yl,
  $C_4$-$C_{13}$-Alk(en)yl Cycloalk(en)yl,
  $C_4$-$C_{13}$-Cycloalk(en)yl Alk(en)yl,
  $C_5$-$C_{13}$-Alk(en)yl Cycloalk(en)yl Alk(en)yl,
  $C_7$-$C_{13}$-Alk(en)yl Bicycloalk(en)yl,
  $C_7$-$C_{13}$-Bicycloalk(en)yl verknüpft,
  $C_8$-$C_{13}$-Alk(en)yl Bicycloalk(en)yl verknüpft,
  $C_8$-$C_{13}$-Bicycloalk(en)yl verknüpft Alk(en)yl,
  $C_9$-$C_{13}$-Alk(en)yl Bicycloalk(en)yl verknüpft Alk(en)yl,
  $C_{10}$-$C_{13}$-Tricycloalk(en)yl verknüpft,
  $C_{11}$-$C_{13}$-Alk(en)yl Tricycloalk(en)yl verknüpft,
  $C_{11}$-$C_{13}$-Tricycloalk(en)yl verknüpft Alk(en)yl
  und $C_{13}$ Alk(en)yl Tricycloalk(en)yl verknüpft Alk(en)yl,

- $R_3$ in einer der tautomeren Formen des Moleküls ein Wasserstoffatom ist,
- $R_4$ ein Wasserstoffatom ist.

5. Verfahren nach Anspruch 1, dadurch gekennzeichnet, daß man eine Verbindung mit der Formel

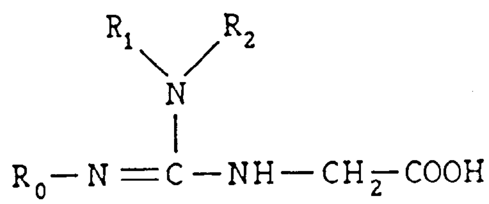

herstellt, worin

- $R_0$ ausgewählt wird unter den Gruppen 2-Cyanopyrid-5-yl, 2-Cyanopyrimidin-5-yl, Indazol-6-yl, Benzisoxazol-5-yl, Benzisoxazol-6-yl, Benzofurazan-5-yl, Isochinol-6-yl, Chinazolin-7-yl, 1,7-Naphtyridin-3-yl oder 1,3,5-Triazanapht-7-yl,
- $R_1$ H oder $CH_3$ ist,
- $R_2$ c-$C_8H_{15}$, $CH_2C_6H_5$, $CH_2$-c-$C_6H_{11}$, $CH(CH_3)C_6H_5$ oder $CH(CH_3)$-c-$C_6H_{11}$.

6. Verfahren nach Anspruch 1, dadurch gekennzeichnet, daß man durch die Reaktion eines Isothiocyanates mit einem Amin gemäß einer der folgenden Reaktionen

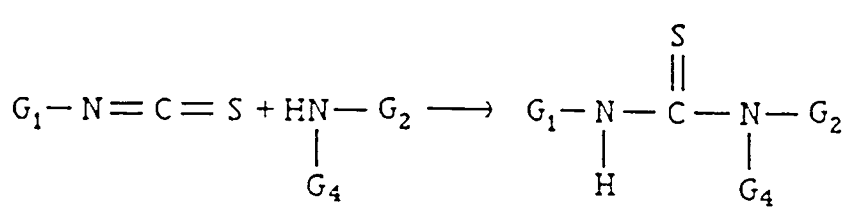

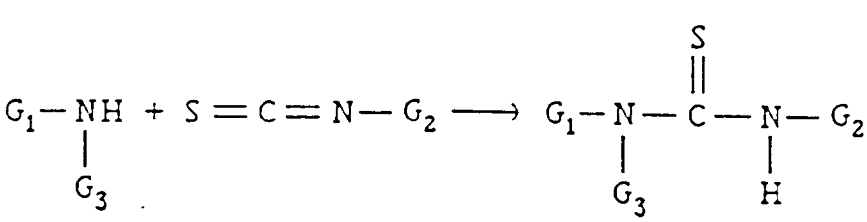

das Thioharnstoffderivat erhält.

7. Verfahren nach Anspruch 1, dadurch gekennzeichnet, daß man das Schwefelatom des Thioharnstoffderivats oder Thioamidoderivats aktiviert, indem man dieses in eine S-Alkylgruppe oder eine $SO_3H$-Gruppe überführt oder indem man es durch eine O-Alkylgruppe, O-$SO_2$-Arylgruppe oder ein Halogenatom ersetzt.

8. Verfahren nach den Ansprüchen 1 und 7, dadurch gekennzeichnet, daß man das Thioharnstoffderivat oder Thioamidoderivat aktiviert, indem man es durch Reaktion mit einem Alkylierungsmittel, das ausgewählt wird unter Methyliodid und Dimethylsulfat, in ein S-Methylisothioharnstoffderivat oder S-Methylisothioamidoderivat mit der allgemeinen Formel

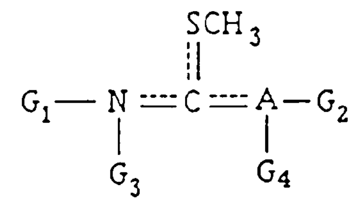

überführt.

9. Verfahren nach Anspruch 8, dadurch gekennzeichnet, daß das S-Methylisothioharnstoffderivat die allgemeine Formel

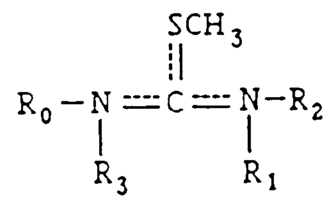

besitzt.

10. Verfahren nach Anspruch 1, dadurch gekennzeichnet, daß man das Thioharnstoffderivat, wenn A = N ist und wenn $G_3$ und $G_4$ ein Wasserstoffatom sind, aktiviert, indem man dieses durch Behandlung mit Phosgen oder durch Reaktion mit einem äquimolaren Gemisch von $(C_6H_5)_3P$ - $CCl_4$ - $(C_2H_5)_3N$ in ein intermediäres Carbodiimid überführt:

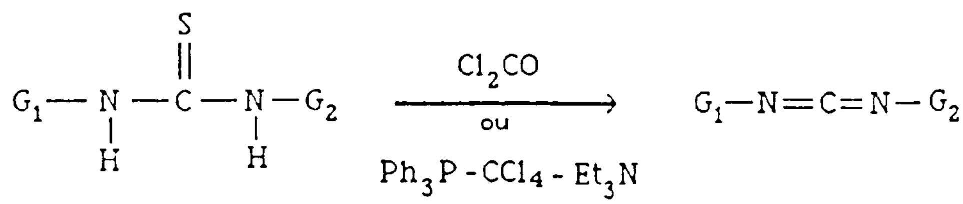


11. Verfahren nach Anspruch 1, dadurch gekennzeichnet, daß, wenn A und A' = N sind und mindestens einer der Reste $G_3$, $G_4$ oder $G_5$ ein Wasserstoffatom ist, das Thioharnstoffderivat mit dem Amin durch Reaktion mit Dicyclohexylcarbodiimid (DCC) als Kupplungsreagens gemäß der folgenden Reaktion kondensiert wird:

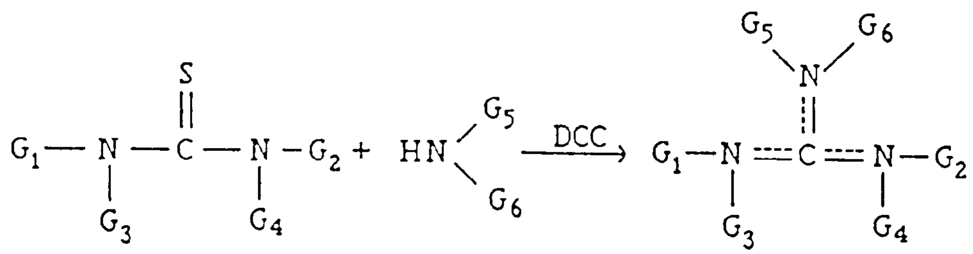

## Claims

### Claims for the following Contracting States : AT, BE, CH, LI, DE, FR, GB, IT, LU, NL, SE

1. Sweetening agents having the general formula :

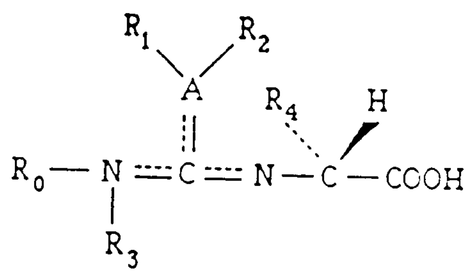

including the tautomeric forms and the physiologically acceptable salts thereof,

– wherein A is selected from the group consisting of N and C ;

– wherein $R_1$ is a hydrogen atom or a hydrocarbon group or a modified hydrocarbon group which contain up to 4 carbon atoms and which can be saturated or unsaturated, acyclic, cyclic or mixed, and wherein, in the modified hydrocarbon group :

. 1 to 2 carbon atoms can be replaced by 1 to 2 heteroatoms, which may be identical or different, selected from the group consisting of nitrogen, oxygen, sulfur, chlorine, bromine and iodine atoms,

. and 1 to 3 hydrogen atoms can be replaced by 1 to 3 fluorine atoms;

– wherein $R_2$ is a hydrocarbon group or a modified hydrocarbon group which contain 2 to 13 carbon atoms and which can be saturated or unsaturated, acyclic, cyclic or mixed, and wherein, in the modified hydrocarbon group :

. 1 to 4 carbon atoms can be replaced by 1 to 4 heteroatoms, which may be identical or different, selected from the group consisting of nitrogen, oxygen, sulfur, chlorine, bromine and iodine atoms,

. and 1 to 5 hydrogen atoms can be replaced by 1 to 5 fluorine atoms ;

– wherein $R_1$ and $R_2$ can be fused ;

– wherein $R_3$ is selected from the group consisting of a hydrogen atom or a $C_1$-$C_3$ alkyl group ;

– wherein $R_4$ is selected from the group consisting of :

H and

CH3 ;

characterized in that $R_0$ is selected from the group consisting of :

. the heterocyclic groups derived from the 1,3-cyclopentadien-1-yl, 2,4-cyclopentadien-1-yl and phenyl parent hydrocarbon groups by replacement of 1 to 3 carbon atoms of the hydrocarbon parent ring by 1 to 3 nitrogen, oxygen and sulfur atoms,

and wherein the hydrogen atoms of the 3- and 4-positions of the 1,3-cyclopentadien-1-yl and 2,4-cyclopentadien-1-yl parent groups and of the 3-, 4- and 5-positions of the phenyl parent group can be substituted in the corresponding heterocyclic group by Br, $CF_3$, $CH_3$, Cl, CN, F, I, $NH_2$, $NO_2$, $OCH_3$ or OH,

. the indan-5-yl, inden-2-yl, inden-5-yl and inden-6-yl carbocyclic groups and the corresponding heterocyclic groups obtained by replacement of 1 to 4 carbon atoms of the hydrocarbon parent ring by 1 to 4 nitrogen, oxygen or sulfur atoms or the SO, CO and $SO_2$ groups for the 1- and 3-carbons of the indan-5-yl parent group, or by the NO or $CCH_3$ groups for the 3-carbon of the 1H-inden-5-yl or for the 1- and 3-carbons of the 2H-inden-5-yl parent group,

and wherein the hydrogen atoms of the 7-position of the indan(or inden)-5-yl and of the 4-position of the inden-6-yl groups can be substituted in the carbocyclic group and the corresponding heterocyclic group by Br, $CF_3$, $CH_3$, Cl, CN, F, I, $NH_2$, $NO_2$, $OCH_3$ or OH,

. the 2-naphthyl and 5,6,7,8-tetrahydro-2-naphthyl carbocyclic groups and the corresponding heterocyclic groups obtained by replacement of 1 to 4 carbon atoms of the hydrocarbon parent ring by 1 to 4 nitrogen, oxygen or sulfur atoms or by the SO, CO and $SO_2$ groups for the 5- and 8-carbons of the 5,6,7,8-tetrahydronaphthyl parent group,

and wherein the hydrogen atom of the 4-position of the 2-naphthyl and 5,6,7,8-tetrahydro-2-naphthyl groups can be substituted, both in the carbocyclic group and in the corresponding heterocyclic group by Br, $CF_3$, $CH_3$, Cl, CN, F, I, $NH_2$, $NO_2$, $OCH_3$ or OH ;

– with the proviso, when A = N, that $R_2$ is not a group

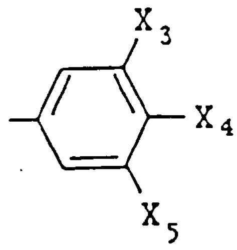

in which $X_3$, $X_4$ and $X_5$ are :

H,
Br,
$CF_3$,
$CF_2CF_3$,
$CH_2CF_3$,
$C_1$-$C_4$ alkyl,
$CH{=}NOCH_3$,
$CH{=}NOH$,
CHO,
$CH_2OCH_3$,
$CH_2OH$,
Cl,
CN,
$COCF_3$,
$COC_1$-$C_3$ alkyl,
$CONH_2$,
$CONHC_1$-$C_3$ alkyl,
$CON(C_1$-$C_3$ alkyl$)_2$,
$COOC_1$-$C_3$ alkyl,
COOH,
F,
I,
$NH_2$,
$NHC_1$-$C_3$ alkyl,
$N(C_1$-$C_3$ alkyl$)_2$,
NHCHO,
$NHCOCH_3$,
$NHCONH_2$,
$NHSO_2CH_3$,
$NO_2$,
$OC_1$-$C_3$ alkyl,
$OCOCH_3$,
OH,
$SC_1$-$C_3$ alkyl,
$SOC_1$-$C_3$ alkyl,
$SO_2C_1$-$C_3$ alkyl,
$SO_2NH_2$,
$SO_2NHC_1$-$C_3$ alkyl,
$SO_2N(C_1$-$C_3$ alkyl$)_2$ and
$SO_3H$.

2. Sweetening agents according to claim 1, characterized in that the heteroatoms, which in the parent hydrocarbon rings replace the carbon atoms to form a corresponding heterocyclic group $R_0$, are :

. N to replace CH, and
. NH, O, S, SO and $SO_2$ to replace $CH_2$.

3. Sweetening agents according to claim 2, characterized in that $R_0$, when $R_0$ is a heterocyclic group, is selected from the group consisting of a pyrrolyl, pyrazolyl, imidazolyl, furyl, oxazolyl, isoxazolyl, oxadiazolyl, furazanyl, thienyl, thiazolyl, isothiazolyl, pyridyl, 2-cyanopyridyl, pyridazinyl, pyrimidinyl, 2-cyanopyrimidinyl,

pyrazinyl, indolyl, isoindolyl, indolizinyl, indazolyl, purinyl, isobenzofuranyl, 3,4-methylenedioxyphenyl, benzisoxazolyl, benzofurazanyl, benzothiazolyl, saccharin-5-yl, saccharin-6-yl, quinolyl, isoquinolyl, cinnolinyl, phthalazinyl, quinazolinyl, quinoxalinyl, naphthyridinyl, 1,5-naphthyridinyl, 1,6-naphthyridinyl, 1,7-naphthyridinyl, 1,3,5-triazanaphthyl, 1,4,5-triazanaphthyl, 1,2,8-triazanaphthyl, 1,3,8-triazanaphthyl, 1,2,5-triazanaphthyl, pteridinyl or isocoumarinyl ring.

4. Sweetening agents according to claim 1, characterized in that A is a nitrogen atom.

5. Sweetening agents according to one of claims 1 to 4, characterized in that $R_1$ is selected from the group consisting of H and $CH_3$.

6. Sweetening agents according to one of claims 1 to 5, characterized in that $R_2$ is selected from the group consisting of :

normal alk(en) (yn)yl $C_2$-$C_{13}$,
branched alk(en) (yn)yl $C_3$-$C_{13}$,
cycloalk(en)yl $C_3$-$C_{13}$,
alk(en)yl cycloalk(en)yl $C_4$-$C_{13}$,
cycloalk(en)yl alk(en)yl $C_4$-$C_{13}$,
alk(en)yl cycloalk(en)yl alk(en)yl $C_5$-$C_{13}$,
alk(en)yl bicycloalk(en)yl $C_7$-$C_{13}$,
fused bicycloalk(en)yl $C_7$-$C_{13}$,
alk(en)yl fused bicycloalk(en)yl $C_8$-$C_{13}$,
fused bicycloalk(en)yl alk(en)yl $C_8$-$C_{13}$,
alk(en)yl fused bicycloalk(en)yl alk(en)yl $C_9$-$C_{13}$,
fused tricycloalk(en)yl $C_{10}$-$C_{13}$,
alk(en)yl fused tricycloalk(en)yl $C_{11}$-$C_{13}$,
fused tricycloalk(en)yl alk(en)yl $C_{11}$-$C_{13}$,
and alk(en)yl fused tricycloalk(en)yl alk(en)yl $C_{13}$,

7. Sweetening agents according to claim 6, characterized in that $R_2$ is a modified hydrocarbon group in which :

. up to 4 carbon atoms can be replaced by heteroatoms, which may be the same or different, selected from the group consisting of :

S to replace C or $CH_2$,
N to replace CH,
NH and O to replace $CH_2$, and
Cl, Br and I to replace $CH_3$,

. and up to 5 hydrogen atoms can be replaced by fluorine atoms.

8. Sweetening agents according to one of claims 1 to 7, characterized in that $R_3$, in one of the tautomeric forms of the molecule, is a hydrogen atom.

9. Sweetening agents according to one of claims 1 to 8, characterized in that $R_4$ is a hydrogen atom.

10. Sweetening agents according to claim 1, characterized in that they consist of compounds of formula :

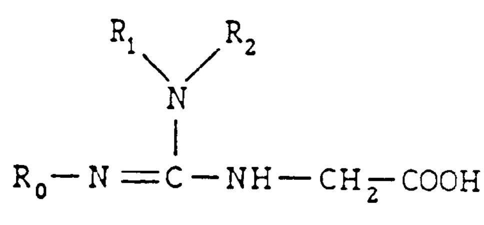

11. Sweetening agents according to claim 10, characterized in that $R_1$ is H or $CH_3$.

12. Sweetening agents according to claims 10 and 11, characterized in that $R_2$ is c-$C_8H_{15}$, $CH_2C_6H_5$, $CH_2$-c-$C_6H_{11}$, $CH(CH_3)C_6H_5$ or $CH(CH_3)$-c-$C_6H_{11}$.

13. Sweetening agents according to claims 10, 11 and 12, characterized in that $R_0$ is selected from the group consisting of a 2-cyanopyrid-5-yl, 2-cyanopyrimidin-5-yl, indazol-6-yl, benzisoxazol-5-yl, benzisoxazol-6-yl, benzofurazan-5-yl, isoquinol-6-yl, quinazolin-7-yl, 1,7-naphthyridin-3-yl or 1,3,5-triazanaphth-7-yl group.

14. Sweetening agents according to claims 1 to 13, characterized in that these compounds are converted to salts by physiologically acceptable inorganic or organic acids or bases.

15. Sweetening agents according to claim 14, characterized in that these compounds are converted to salts in the form of hydrochlorides or of sodium, potassium, ammonium, calcium and magnesium salts.

16. Process for sweetening the foods, beverages, confectionaries, pastries, chewing gum, hygiene products, cosmetics, toiletries, pharmaceutical and veterinary products characterized in that it consists in adding

to these products an adequate quantity of at least one sweetening agent according to one of claims 1 to 15.

17. Preparations which have been sweetened according to the process of claim 16.

18. Sweetening compositions characterized in that they consist of an adequate quantity of one or more sweetening agents according to one of claims 1 to 15 and a carrier or bulking agent selected from the group consisting of polydextrose, starch, maltodextrins, cellulose , methylcellulose, carboxymethylcellulose, hydroxymethylcellulose, microcrystalline cellulose, sodium alginate, pectins, gums, lactose, maltose, glucose, leucine, glycerol, mannitol, sorbitol, sodium bicarbonate and phosphoric, citric, tartaric, fumaric, benzoic, sorbic and propionic acids, and their sodium, potassium and calcium salts.

19. Sweetening compositions characterized in that they consist of a sweetening agent according to one of claims 1 to 15 and at least one other sweetening agent, the other sweetening agent being selected from the group consisting of sucrose, corn syrup, fructose, aspartame, alitame, neohesperidin dihydiochalcone, hydrogenated isomaltulose, stevioside, L-sugars, glycyrrhizin, xylitol, sorbitol, mannitol, acesulfame-K, saccharin and its sodium, potassium, ammonium or calcium salts, cyclamic acid and its sodium, potassium or calcium salts, trichlorogalactosucrose, monellin and thaumatin

**Claims for the following Contracting States : ES, GR**

1. Process for preparation of a sweetening agent of general formula :

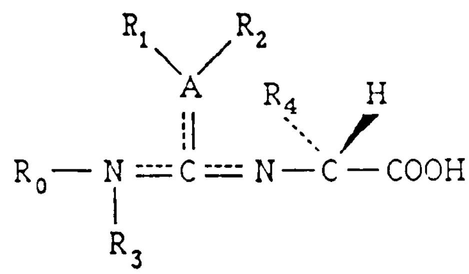

including the tautomeric forms and the physiologically acceptable salts thereof,

– wherein $R_0$ is selected from the group consisting of :

. the heterocyclic groups derived from the 1,3-cyclopentadien-1-yl, 2,4-cyclopentadien-1-yl and phenyl parent hydrocarbon groups by replacement of 1 to 3 carbon atoms of the hydrocarbon parent ring by 1 to 3 nitrogen, oxygen and sulfur atoms,

and wherein the hydrogen atoms of the 3- and 4-positions of the 1,3-cyclopentadien-1-yl and 2,4-cyclopentadien-1-yl parent groups and of the 3-, 4- and 5-positions of the phenyl parent group can be substituted in the corresponding heterocyclic group by Br, $CF_3$, $CH_3$, Cl, CN, F, I, $NH_2$, $NO_2$, $OCH_3$ or OH,

. the indan-5-yl, inden-2-yl, inden-5-yl and inden-6-yl carbocyclic groups and the corresponding heterocyclic groups obtained by replacement of 1 to 4 carbon atoms of the hydrocarbon parent ring by 1 to 4 nitrogen, oxygen or sulfur atoms or the SO, CO and $SO_2$ groups for the 1- and 3-carbons of the indan-5-yl parent group, or by the NO or $CCH_3$ groups for the 3-carbon of the 1H-inden-5-yl or for the 1- and 3-carbons of the 2H-inden-5-yl parent group,

and wherein the hydrogen atoms of the 7-position of the indan(or inden)-5-yl and of the 4-position of the inden-6-yl groups can be substituted in the carbocyclic group and the corresponding heterocyclic group by Br, $CF_3$, $CH_3$, Cl, CN, F, I, $NH_2$, $NO_2$, $OCH_3$ or OH,

. the 2-naphthyl and 5,6,7,8-tetrahydro-2-naphthyl carbocyclic groups and the corresponding heterocyclic groups obtained by replacement of 1 to 4 carbon atoms of the hydrocarbon parent ring by 1 to 4 nitrogen, oxygen or sulfur atoms or by the SO, CO and $SO_2$ groups for the 5- and 8-carbons of the 5,6,7,8-tetrahydronaphthyl parent group,

and wherein the hydrogen atom of the 4-position of the 2-naphthyl and 5,6,7,8-tetrahydro-2-naphthyl groups can be substituted, both in the carbocyclic group and in the corresponding heterocyclic group by Br, $CF_3$, $CH_3$, Cl, CN, F, I, $NH_2$, $NO_2$, $OCH_3$ or OH ;

– with the proviso, when A = N, that $R_2$ is not a group

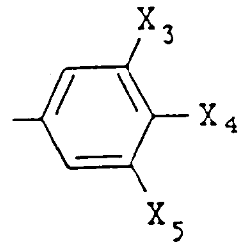

in which $X_3$, $X_4$ and $X_5$ are :

H,
Br,
$CF_3$,
$CF_2CF_3$,
$CH_2CF_3$,
$C_1$-$C_4$ alkyl,
$CH{=}NOCH_3$,
CH=NOH,
CHO,
$CH_2OCH_3$,
$CH_2OH$,
Cl,
CN,
$COCF_3$,
$COC_1$-$C_3$ alkyl,
$CONH_2$,
$CONHC_1$-$C_3$ alkyl,
$CON(C_1$-$C_3$ alkyl$)_2$,
$COOC_1$-$C_3$ alkyl,
COOH,
F,
I,
$NH_2$,
$NHC_1$-$C_3$ alkyl,
$N(C_1$-$C_3$ alkyl$)_2$,
NHCHO,
$NHCOCH_3$,
$NHCONH_2$,
$NHSO_2CH_3$,
$NO_2$,
$OC_1$-$C_3$ alkyl,
$OCOCH_3$,
OH,
$SC_1$-$C_3$ alkyl,
$SOC_1$-$C_3$ alkyl,
$SO_2C_1$-$C_3$ alkyl,
$SO_2NH_2$,
$SO_2NHC_1$-$C_3$ alkyl,
$SO_2N(C_1$-$C_3$ alkyl$)_2$ and
$SO_3H$ ;

– wherein A is selected from the group consisting of N and C ;

– wherein $R_1$ is a hydrogen atom or a hydrocarbon group or a modified hydrocarbon group which contain up to 4 carbon atoms and which can be saturated or unsaturated, acyclic, cyclic or mixed, and wherein, in the modified hydrocarbon group :

. 1 to 2 carbon atoms can be replaced by 1 to 2 heteroatoms, which may be identical or different, selected from the group consisting of nitrogen, oxygen, sulfur, chlorine, bromine and iodine atoms,

. and 1 to 3 hydrogen atoms can be replaced by 1 to 3 fluorine atoms;

– wherein $R_2$ is a hydrocarbon group or a modified hydrocarbon group which contain 2 to 13 carbon atoms and which can be saturated or unsaturated, acyclic, cyclic or mixed, and wherein, in the modified hydrocarbon group :

. 1 to 4 carbon atoms can be replaced by 1 to 4 heteroatoms, which may be identical or different, selected from the group consisting of nitrogen, oxygen, sulfur, chlorine, bromine and iodine atoms,

. and 1 to 5 hydrogen atoms can be replaced by 1 to 5 fluorine atoms ;

– wherein $R_1$ and $R_2$ can be fused ;

– wherein $R_3$ is selected from the group consisting of a hydrogen atom or a $C_1$-$C_3$ alkyl group ;

– wherein $R_4$ is selected from the group consisting of :

H and

$CH_3$ ;

characterized in that it consists in condensing a thioureido or thioamido derivative of general formula :

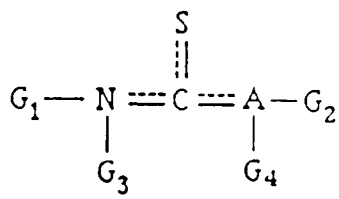

with a compound of general formula :

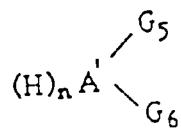

wherein :

. $G_1$ is $R_0$ or $HOOCCHR_4$,

. $G_2$ is $R_2$ or $HOOCCHR_4$,

. $G_3$, $G_4$ and $G_5$ are H, $R_1$ or $R_3$,

. $G_6$ is $R_0$, $R_2$ or $HOOCCHR_4$,

. A′ is N or C,

. n is equal to 1 when A′ is N, and is equal to 2 when A′ is C ;

and wherein :

. $G_1$, $G_2$ and $G_6$, are not simultaneously identical,

. A and A′ are not simultaneously carbon atoms,

. A′ is N and $G_3$, $G_4$, and $G_5$ are H when $G_1$ or $G_2$ or $G_6$ are $HOOCCHR_4$;

and wherein $R_0$, $R_1$, $R_2$, $R_3$, $R_4$ and A correspond to the definitions given hereinbefore.

2. Process according to claim 1, characterized in that the heteroatoms, which in the parent hydrocarbon rings replace the carbon atoms to form a corresponding heterocyclic group $R_0$, are :

. N to replace CH, and

. NH, O, S, SO and $SO_2$ to replace $CH_2$.

3. Process according to claim 1, characterized in that $R_0$, when $R_0$ is a heterocyclic group, is selected from the group consisting of a pyrrolyl, pyrazolyl, imidazolyl, furyl, oxazolyl, isoxazolyl, oxadiazolyl, furazanyl, thienyl, thiazolyl, isothiazolyl, pyridyl, 2-cyanopyridyl, pyridazinyl, pyrimidinyl, 2-cyanopyrimidinyl, pyrazinyl, indolyl, isoindolyl, indolizinyl, indazolyl, purinyl, isobenzofuranyl, 3,4-methylenedioxyphenyl, benzisoxazolyl, benzofurazanyl, benzothiazolyl, saccharin-5-yl, saccharin-6-yl, quinolyl, isoquinolyl, cinnolinyl, phthalazinyl, quinazolinyl, quinoxalinyl, naphthyridinyl, 1,5-naphthyridinyl, 1,6-naphthyridinyl, 1,7-naphthyridinyl, 1,3,5-triazanaphthyl, 1,4,5-triazanaphthyl, 1,2,8-triazanaphthyl, 1,3,8-triazanaphthyl, 1,2,5-triazanaphthyl, pteridinyl or isocoumarinyl ring.

4. Process according to claim 1, characterized in that :

– A is a nitrogen atom;

– $R_1$ is selected from the group consisting of H and $CH_3$;

– $R_2$ is selected from the group consisting of :

normal alk(en) (yn)yl $C_2$-$C_{13}$,

branched alk(en) (yn)yl $C_3$-$C_{13}$,

cycloalk(en)yl $C_3$-$C_{13}$,
alk(en)yl cycloalk(en)yl $C_4$-$C_{13}$,
cycloalk(en)yl alk(en)yl $C_4$-$C_{13}$,
alk(en)yl cycloalk(en)yl alk(en)yl $C_5$-$C_{13}$,
alk(en)yl bicycloalk(en)yl $C_7$-$C_{13}$,
fused bicycloalk(en)yl $C_7$-$C_{13}$,
alk(en)yl fused bicycloalk(en)yl $C_8$-$C_{13}$,
fused bicycloalk(en)yl alk(en)yl $C_8$-$C_{13}$,
alk(en)yl fused bicycloalk(en)yl alk(en)yl $C_9$-$C_{13}$;
fused tricycloalk(en)yl $C_{10}$-$C_{13}$,
alk(en)yl fused tricycloalk(en)yl $C_{11}$-$C_{13}$,
fused tricycloalk(en)yl alk(en)yl $C_{11}$-$C_{13}$,
and alk(en)yl fused tricycloalk(en)yl alk(en)yl $C_{13}$;

– $R_3$, in one of the tautomeric forms of the molecule, is a hydrogen atom;
– $R_4$ is a hydrogen atom.

5. Process according to claim 1, characterized in that a compound of formula is prepared :

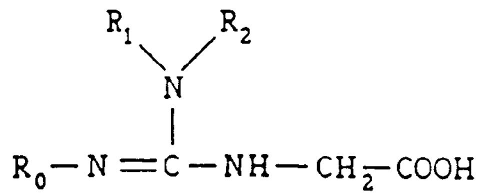

wherein :

– $R_0$ is selected from the group consisting of a 2-cyanopyrid-5-yl, 2-cyanopyrimidin-5-yl, indazol-6-yl, benzisoxazol-5-yl, benzisoxazol-6-yl, benzofurazan-5-yl, isoquinol-6-yl, quinazolin-7-yl, 1,7-naphthyridin-3-yl or 1,3,5-triazanaphth-7-yl group,
– $R_1$ is H or $CH_3$,
– $R_2$ is c-$C_8H_{15}$, $CH_2C_6H_5$, $CH_2$-c-$C_6H_{11}$, $CH(CH_3)C_6H_5$ or $CH(CH_3)$-c-$C_6H_{11}$.

6. Process according to claim 1, characterized in that it consists in obtaining the thioureido derivative by reaction of an isothiocyanate with an amine according to one of the reactions :

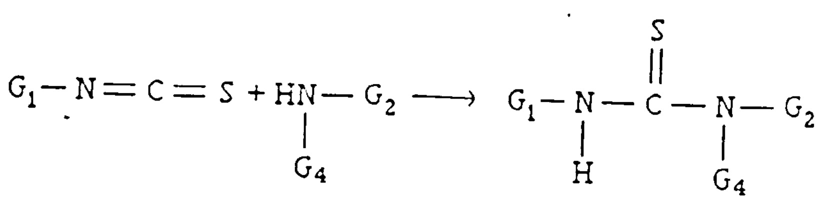

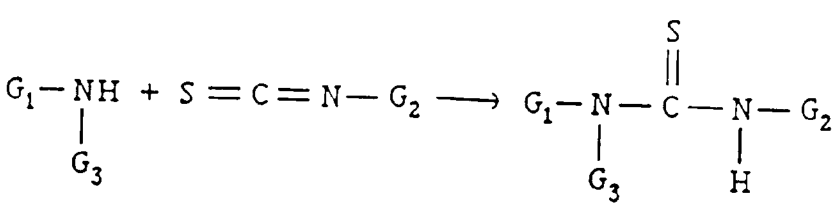

7. Process according to claim 1, characterized in that it consists in activating the sulfur atom of the thioureido or thioamido derivative by transforming it to a S-alkyl or $SO_3H$ group, or by replacing it by an O-alkyl or O-$SO_2$aryl group or a halogen atom.

8. Process according to claims 1 and 7, characterized in that it consists in activating the thioureido or thioamido derivative by transforming it to a S-methylisothioureido or S-methylisothioamido derivative of general formula:

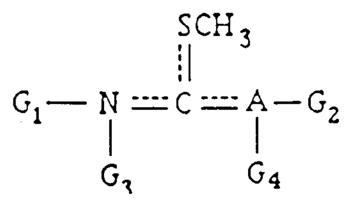

by the action of an alkylating agent selected from the group consisting of methyl iodide and dimethyl sulfate.

9. Process according to claim 8, characterized in that the S-methylisothioureido derivative corresponds to the general formula:

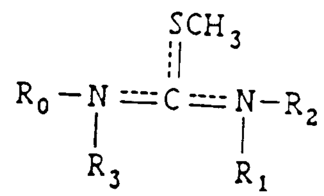

10. Process according to claim 1, characterized in that it consists in activating the thioureido derivative when A is N and when $G_3$ and $G_4$ are hydrogen atoms, by transformation to a carbodiimide intermediate :

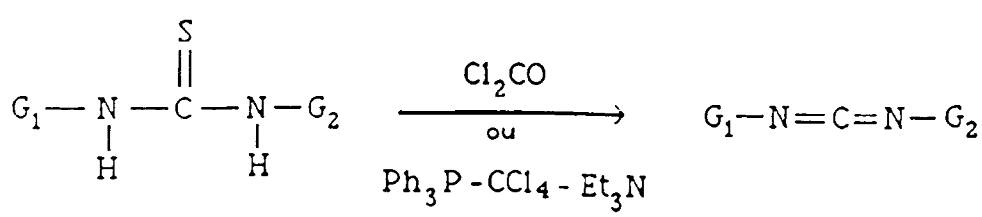


either by treatment by phosgene or by the action of an equimolecular mixture of $(C_6H_5)_3P$ - $CCl_4$ - $(C_2H_5)_3N$.

11. Process according to claim 1, characterized in that when A and A' are N and when at least one of the $G_3$, $G_4$ or $G_5$ radicals is a hydrogen atom, the condensation of the thioureido derivative with the amine is realized by action of dicyclohexylcarbodiimide (DDC) as coupling agent, according to the reaction :